# FASCICULE DE BREVET EUROPEEN

(11) **EP 0 451 049 B1**
(45) Date de publication et mention de la délivrance du brevet: **21.10.1998**
(21) Numéro de dépôt: 91400897.4
(22) Date de dépôt: 02.04.1991
(51) Int. Cl.: G06F 17/30

(54) **Procédé et système de stockage et de recherche de formules chimiques dans une base de données**
Verfahren und Speicher- und Abrufsystem von chemischen Formeln in einer Datenbank
Method and storage/retrieval system of chemical formulae in a database

(30) Priorité: 30.03.1990 FR 9004134
(43) Date de publication de la demande: 09.10.1991
(73) Titulaire: QUESTEL, 75013 Paris (FR)
(72) Inventeur: Renaud, Dominique, F-95600 Eaubonne (FR); Roussel, Jean-Claude, F-92400 Courbevoie (FR)
(74) Mandataire: Gutmann, Ernest

(56) Documents cités:
- EP-A- 0 196 237
- US-A- 4 642 762
- J. CHEM. INF. COMPUT. SCI., vol. 21, 1981, pages 148-150, ACS, Washington, US; M. LYNCH et al.: "Computer storage and retrieval of generic chemical structures in patents. 1. Introduction and general strategy"
- J. CHEM. INF. COMPUT. SCI., vol. 24, 1984, pages 57-66, ACS, Washington, US; S. WELFORD et al.: "Computer storage and retrieval of generic chemical structures in patents. 5. Algorithmic generation of fragment descriptors for generic structure screening"
- J. OF CHEM. DOCUMENTATION, vol. 13, no. 1, 1973, pages 8-13, ACS, Washington, US; J. DUBOIS: "French national policy for chemical information and the DARC system as a potential tool of this policy"

## Description

La présente invention concerne un procédé et un système de stockage et de recherche dans une base de données de formules de Markush, et d'informations associées auxdites formules.

Une formule de Markush est une représentation structurelle d'un composant ou d'une famille de composants chimiques ayant des caractéristiques structurelles communes. Une telle formule est représentée par un schéma comprenant une partie commune à la famille de composants, ou groupe de base, et une ou plusieurs parties de caractéristiques différentes, ou groupes "substituants", habituellement représentés par des symboles tels que R, R₁, etc...

L'invention trouve une application particulièrement importante bien que non exclusive dans les domaines de la recherche pharmaceutique et de la chimie industrielle. Elle permet le stockage, la recherche et l'identification d'informations sur des formules de Markush mentionnées dans la littérature et en particulier dans les revendications de brevet.

L'invention peut ainsi être utilisée dans l'étude des propriétés d'une molécule, ou d'une famille de molécules, destinées par exemple à un médicament ou à un produit chimique industriel.

On connaît depuis le début des années 1960, des procédés et dispositifs automatisés de stockage et de recherche d'informations sur des formules chimiques, atome par atome.

Pratiquer d'entrée de jeu une telle recherche sur des formules simples, est possible.

L'appliquer aux formules de Markush, dont une simple formule peut représenter plusieurs millions de composants, n'est pas satisfaisant, car trop long.

On a donc cherché des procédés plus performants en prévoyant une première élimination de formules possibles, avant d'effectuer la recherche atome par atome.

Le brevet US 4.642.762 décrit un procédé de stockage et de recherche de formules de Markush comportant une étape de présélection de ce type. Pour ce faire, on commence par réécrire les formules de Markush sous la forme d'une représentation multiple spécifique, puis sous la forme d'une représentation multiple générique correspondante, représentant toutes les représentations individuelles génériques desdites formules de Markush.

On compare ensuite les représentations individuelles génériques de la formule recherchée avec les représentations individuelles génériques des formules stoquées.

Par représentation générique il faut entendre, dans le brevet US 4.642.762, des représentations dans lesquelles figurent uniquement des termes dit générique du type alkyle, carboxyle, etc...

Il est annoncé qu'on peut ainsi éliminer partiellement un certain nombre de formules de Markush possibles de la banque de données, dès lors que celles-ci ne sont pas représentables sous les formes individuelles génériques compatibles avec celles de la formule recherchée.

Un tel procédé n'est pas entièrement satisfaisant et peut être amélioré. En particulier, en élargissant la représentation de la formule de Markush recherchée pour la présenter sous une forme dite "générique", on crée des possibilités supplémentaires de correspondance, qui diminuent la précision de la recherche, rendant le gain de traitement en temps moins important, voir discutable.

La présente invention vise à fournir un procédé et un système de stockage et de recherche de formules chimiques répondant mieux que ceux antérieurement connus aux exigences de la pratique notamment en ce qu'ils permettent de rechercher dans un fichier de formules de Markush préalablement constitué, une formule déterminée avec un haut degré de fiabilité et une grande rapidité.

L'invention permet d'obtenir directement les images des formules chimiques proches de, ou correspondant à, la formule chimique recherchée de façon automatique ou semi-automatique et sous une forme modifiée et améliorée. Une comparaison aisée par l'utilisateur, de l'image de la formule recherchée, avec celle des formules obtenues par la recherche, va, par ailleurs, être possible.

L'invention, qui pose et résout le problème technique complexe qui consiste à retrouver, parmi une quantité extrêmement importante de formules chimiques figurant dans la littérature sous de multiples formes et représentations différentes, des éléments et informations concernant une formule spécifique, permet d'effectuer cette recherche de façon interactive avec l'opérateur. Elle permet ainsi, comme on va le voir, de faire fonctionner le système informatique mis en oeuvre avec l'invention, avec un rendement optimal, notamment en temps d'utilisation de son calculateur, ce qui autorise, à puissance égale, un plus grand nombre d'utilisateurs simultanés. L'optimisation de la recherche, grâce à l'invention, fait ainsi gagner un temps important de branchement entre l'utilisateur et le système informatique, et permet donc de diminuer les coûts.

Le procédé et le système de l'invention sont, par ailleurs, mis en oeuvre de façon simple et aisée par l'utilisateur du système".

Dans ce but la présente invention propose notamment un procédé de stockage et de recherche de formules de Markush caractérisé en ce que
a) on forme un fichier de base de formules chimiques à partir desdites formules de Markush, dans lequel chaque formule de Markush est stockée sous forme de table de connectivité comprenant un numéro de référence de ladite formule stockée et un numéro de "présélection" décrivant synthétiquement des caractères types prédéterminés de ladite formule, définis selon une table de définition préalablement constituée desdits caractères types,
b) on détermine pour chaque formule du fichier de base et pour la formule recherchée stockée sous forme identique aux formules du fichier de base, la liste de tous les noeuds ou atomes foyers comprenant au moins deux atomes voisins immédiats et, un fragment étant défini comme une partie de structure de formule constituée par un foyer, ses atomes voisins immédiats (environnement 1) et s'ils existent le ou les atomes voisins immédiats (environnement 2) de chacun des atomes voisins immédiats du foyer, on calcule, pour chaque noeud de cette liste, toutes les parties possibles ou fragments de ladite formule issues de ce noeud,
c) on forme un fichier de fragments comprenant, pour chaque fragment calculé des formules du fichier de base, la liste des numéros de références des formules comprenant ce fragment,
d) pour un premier fragment de la formule de Markush recherchée,
   on extrait dudit fichier de fragments une première liste de numéros de référence des formules possibles comprenant ledit fragment de ladite formule de Markush recherchée,
e) puis s'il y a lieu et pour un second fragment de la formule recherchée, on extrait dudit fichier de fragments une deuxième liste de numéros de référence des formules possibles comprenant ledit deuxième fragment, que l'on compare avec la première liste obtenue pour le premier fragment, pour former une troisième liste "issue des fragments", constituée par les numéros communs des deux premières listes, et on réitère l'opération tant qu'on l'estime nécessaire en comparant chaque nouvelle liste de numéros de formules possibles correspondant à un nouveau fragment avec la liste constituée par les numéros communs de toutes les listes précédentes, pour former une liste "issue de fragments" de longueur optimisée,
f) on compare le numéro de "présélection" de la formule de Markush recherchée avec les numéros de "présélection" des formules de la liste "issue de fragments" de longueur optimisée, et on en extrait une liste finale formée par les numéros de références des formules ayant un numéro de "présélection" identique à celui de la formule de Markush recherchée.

L'invention propose également un procédé de stockage et de recherche de formules de Markush (1) par un opérateur, caractérisé en ce que
a) on forme un fichier de base (14) de formules chimiques à partir desdites formules de Markush, dans lequel chaque formule de Markush est stockée dans un système informatique par l'opérateur, sous forme de tables de connectivité (TC₁, TC₂...TCₙ) comprenant un numéro de référence ( NOFM₁, NOFM₂,.. NOFMₙ) de ladite formule stockée et un numéro de "présélection" (Nps₁, Nps₂,.. Npsₙ) décrivant synthétiquement des caractères types prédéterminés de ladite formule, définis selon une table de définition préalablement constituée desdits caractères types (table VII),
b) on détermine automatiquement pour chaque formule du fichier de base (14) et pour la formule recherchée (3) stockée sous forme identique aux formules du fichier par l'opérateur, la liste de tous les noeuds ou atomes foyers comprenant au moins deux voisins immédiats et, un "fragment" étant défini comme une partie de structure de formule constituée par un foyer, ses atomes voisins immédiats (environnement 1) et s'ils existent le ou les atomes voisins immédiats (environnement 2) de chacun des atomes voisins immédiats du foyer, on calcule automatiquement, pour chaque noeud de cette liste, toutes les parties possibles, ou fragments, de ladite formule issues de ce noeud,
c) on forme automatiquement un fichier de fragments (18) comprenant, pour chaque fragment calculé des formules du fichier de base, la liste des numéros de références desdites formules comprenant ce fragment,
d) pour un premier fragment de la formule de Markush recherchée,
   on extrait dudit fichier de fragments (18) une première liste de numéros de référence des formules possibles comprenant ledit fragment de ladite formule de Markush recherchée,
e) puis, s'il y a lieu, et suite à une commande de l'opérateur, et pour un second fragment de la formule recherchée, on extrait dudit fichier de fragments (18), une deuxième liste de numéros de référence des formules possibles comprenant ledit deuxième fragment, que l'on compare automatiquement avec la première liste obtenue pour le premier fragment, pour former une troisième liste "issue des fragments", constituée par les numéros communs des deux premières listes, et on réitère l'opération par commande manuelle de l'opérateur en comparant automatiquement chaque nouvelle liste de numéros de formules possibles correspondant à un nouveau fragment avec la liste constituée par les numéros communs de toutes les listes précédentes, pour former automatiquement une liste "issue de fragments" de longueur optimisée,
f) puis, suite à une commande manuelle de l'opérateur, on compare automatiquement le numéro de "présélection" de la formule de Markush recherchée avec les numéros de "présélection" des formules de la liste "issue de fragments" de longueur optimisée, et on en extrait automatiquement une liste finale formée par les numéros de références des formules ayant un numéro de "présélection" identique à celui de la formule de Markush recherchée.

Dans un autre mode de réalisation, l'invention propose un procédé de stockage et de recherche de formules de Markush (1) par un utilisateur dans un système informatique, caractérisé en ce que,
partant d'une base de données (6) du système informatique comprenant un fichier de base (14) de formules chimiques formé à partir de formules de Markush (1), fichier dans lequel chaque formule de Markush est stockée sous forme de table de connectivité (TCᵢ) avec un numéro de référence (NOFMᵢ) de ladite formule stockée et un numéro de "présélection" (Npsᵢ) décrivant synthétiquement des caractères types prédéterminés de ladite formule définis selon une table de définition préalablement constituée desdits caractères types (table VII),
ladite base (6) comprenant également un fichier de fragments (18) comprenant les listes des numéros de références des formules associées à chaque fragment des formules du fichier de base (14), un fragment étant défini comme une partie de structure de formule constituée par un foyer, ses atomes voisins immédiats (environnement 1) et s'ils existent le ou les atomes voisins immédiats (environnement 2) de chacun des atomes voisins immédiats du foyer,
a) l'utilisateur introduit au moins partiellement la formule de Markush recherchée (3) sous une forme graphique déterminée dans le système informatique,
b) le système informatique lit ladite représentation graphique et la transforme pour la stocker sous forme similaire aux formules de la base de données (6), c'est à dire sous forme de table de connectivité (TCᵢ) avec un numéro de présélection (Npsi) de la dite formule recherchée,
c) le système informatique détermine automatiquement pour la formule recherchée (3), la liste de tous les noeuds comprenant au moins deux voisins immédiats, et calcule, pour chaque noeud de cette liste, tous les fragments de ladite formule issus de ce noeud,
d) pour un premier fragment de la formule de Markush recherchée, le système informatique extrait automatiquement dudit fichier de fragments (18) une première liste de numéros de référence des formules possibles comprenant ledit fragment de ladite formule de Markush recherchée,
e) puis, s'il y a lieu et pour un second fragment de la formule recherchée, le système informatique extrait automatiquement dudit fichier de fragments (18) une deuxième liste de numéros de référence des formules possibles comprenant ledit deuxième fragment, qu'il compare avec la première liste obtenue pour le premier fragment, pour former une troisième liste "issue des fragments", constituée par les numéros communs des deux premières listes,
   sachant que l'opération est réitérée par le système informatique qui compare automatiquement chaque nouvelle liste de numéros de formules possibles correspondant à un nouveau fragment avec la liste constituée par les numéros communs de toutes les listes précédentes, jusqu'à ce que ladite comparaison ne permette plus de réduire le nombre de formules de la liste "issue de fragments" de façon significative, pour former une liste issue de fragments de longueur optimisée,
f) le système informatique, en automatique, ou l'utilisateur, en manuel, commande ensuite une étape où ledit système informatique compare le numéro de "présélection" de la formule de Markush recherchée avec les numéros de "présélection" des formules de la liste "issue de fragments" de longueur optimisée, et en extrait une liste finale formée par les numéros de références des formules ayant un numéro de "présélection" identique à celui de la formule de Markush recherchée,
g) puis, le système informatique extrait, une à une, à la demande de l'utilisateur, et s'il y a lieu, les formules détenues dans ladite liste finale, en les transformant pour les représenter sous forme d'une image graphique adaptée propre à permettre une comparaison optimisée avec la forme graphique déterminée de la formule recherchée.

Dans des modes de réalisation préférés, on a recours en outre à l'une et/ou à l'autre des dispositions suivantes :
- On effectue de plus, une comparaison noeud à noeud des formules de ladite liste finale avec la formule de Markush recherchée, et on extrait les références de la ou des formules de Markush éventuellement obtenues par cette comparaison.
- On affecte à certains des atomes ou groupes d'atomes, des formules de Markush du fichier de base, un ou plusieurs attributs, et on ne recherche dans ledit fichier de base de formules de Markush que les formules comportant une structure munie du ou des attributs spécifiquement affectés aux atomes, ou groupe d'atomes, de la formule recherchée.
- On stocke les formules de Markush, constituées par des graphes, en utilisant quand nécessaire aux noeuds desdits graphes, de "faux atomes" représentant des termes génériques et/ou des ensembles d'atomes invariants pour simplifier les représentations desdites formules.
- On entre les formules de Markush dans le fichier de formules chimiques, et on interroge le fichier, sous forme graphique.
   Ceci permet la visualisation des formules préalablement stockées de façon automatique, notamment lors d'une interrogation.
- On constitue le numéro de "présélection" décrivant synthétiquement des caractères types prédéterminés d'une formule de Markush, sous forme d'une chaîne de n bits, dont chaque bit correspond à un caractère type déterminé de la table de définition préalablement constituée desdits n caractères types.
- On stocke chaque formule de Markush sous forme de table de connectivité "à tiroir", comprenant :
   quatre tables donnant les relations entre les différents groupes d'atomes, ou graphes "fils" de la structure, constituées par une première table (LIRAT) identifiant les atomes auxquels se rattachent lesdits groupes,
   une deuxième table (NVAL) donnant les nombres de valeurs pour chacun desdits groupes,
   une troisième table (IAT) donnant les débuts de valeurs desdits groupes et,
   une quatrième table (IFR) donnant les adresses par groupe vers la table des débuts de valeurs (IAT).

Une telle disposition permet d'améliorer d'un facteur 5 la rapidité d'interrogation du fichier en cas de recherche atome par atome, ce qui revient à étendre virtuellement la mémoire de travail du système informatique interrogé.

L'invention propose également un système ou dispositif informatique pour la mise en oeuvre du procédé de stockage et de recherche de formules de Markush ci-dessus défini, comprenant une unité centrale, des mémoires de travail et de stockage, des moyens d'entrées de formules de Markush sous forme graphique et/ou alpha-numérique et des moyens de restitution d'informations caractérisé en ce qu'il comprend
des moyens pour générer un fichier de base de formules chimiques, à partir desdites formules de Markush, sous forme de tables de connectivité, chaque formule comprenant un numéro de référence et un numéro de "présélection" décrivant des caractères types de ladite formule définis selon une table de définition préalablement stokée dans une mémoire du système,
des moyens pour générer, pour chaque formule stockée ou recherchée, la liste de tous les noeuds, ou atomes foyers, comprenant au moins deux atomes voisins immédiats,
des moyens de calcul, pour chaque noeud de cette liste par formule, de toutes les parties possibles, dites fragments, de ladite formule, issues de ce noeud, un fragment étant défini comme une partie de structure de formule constituée par un foyer, ses atomes voisins immédiats (environnement 1) et s'ils existent le ou les atomes voisins immédiats (environnement 2 ) de chacun des atomes voisins immédiats du foyer,
des moyens pour générer un fichier de fragments comprenant, pour chaque fragment calculé des formules du fichier de base, la liste des numéros de références des formules comprenant ce fragment,
des moyens d'extraction, à partir dudit fichier de fragments, de listes de numéros de référence de formules possibles comprenant au moins un fragment de la formule de Markush recherchée, les dits moyens étant prévus pour générer une liste "issue de fragments" de longueur optimisée constituée par les numéros des formules de Markush comportant un nombre optimisé, déterminé par l'utilisateur, de fragments communs avec la formule de Markush recherchée,
et des moyens de comparaison du numéro de "présélection" de la formule de Markush recherchée, avec les numéros de "présélection" des formules de la liste "issue de fragments" de longueur optimisée et, à partir de cette comparaison, de constitution d'une liste finale formée par les numéros de références des formules ayant un numéro de "présélection" identique à celui de la formule de Markush recherchée.
Le système informatique selon l'invention comprend de plus avantageusement des moyens de comparaison noeud à noeud des formules de ladite liste finale avec la formule de Markush recherchée et d'extraction des références de la ou des formules de Markush éventuellement obtenues par cette comparaison.

L'invention sera mieux comprise à la lecture de la description qui suit de modes particuliers de réalisation de l'invention, donnés à titre d'exemples non limitatifs.

La description se réfère aux dessins qui l'accompagnent, dans lesquels :
- La figure 1 est une représentation graphique classique d'une formule de Markush.
- La figure 2 est une représentation graphique d'une formule de Markush telle qu'utilisée dans l'invention; chaque formule est représentée sous forme d'un graphe "à tiroirs" c'est-à-dire présentant des noeuds connectés à des groupes ou fragments de graphe variables ou "substituants", susceptibles d'avoir eux-mêmes des noeuds connectés à d'autres groupes "substituants".
- La figure 3 est un tableau donnant un choix de symboles représentant certaines parties de graphe ou configurations apparaissant fréquemment dans les formules chimiques, et avantageusement utilisés dans l'invention, pour faciliter stockage et recherche.
- La figure 4 donne un mode préféré de représentation graphique des types de liaison entre atomes tels qu'utilisés dans les représentations graphiques des formules stockées et recherchées par la méthode selon l'invention.
- La figure 5 est un schéma de principe du système de stockage et de recherche de formules de Markush selon l'invention.
- Les figures 6a et 6b présentent les organigrammes schématiques, respectivement de la saisie des formules et de la recherche de formule de Markush, selon le procédé de l'invention.
- Les figures 7a et 7b montrent deux exemples de formule chimique et de leurs fragments, tels que définis et utilisés dans le procédé et le système selon l'invention.
- Les figures 8, 9 et 10 donnent les représentations graphiques successives des groupes et sous-groupes d'une formule particulière,tel que saisies ou éditées en utilisant un mode de réalisation du système et du procédé selon l'invention.
- Les figures 11a et 11b donnent un mode de réalisation de table de connectivité utilisée dans le procédé selon l'invention pour la formule des figures 8, 9 et 10.
- La figure 12 est un exemple de tableau d'attributs, selon un mode de réalisation de l'invention, permettant de préciser les atomes et les liaisons des formules de Markush et accélérer la recherche. L'exemple correspond à la formule des figures 8, 9, 10, 11a et 11b.
- La figure 13 représente un mode de réalisation schématique d'un système de stockage et de recherche de formule de Markush, selon l'invention.

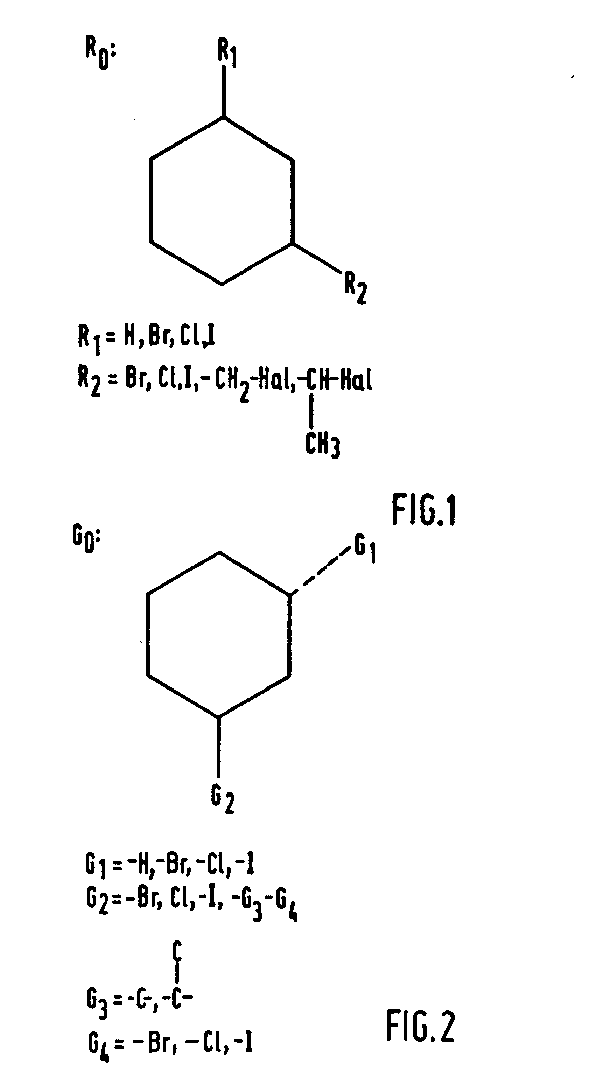

La figure 1 est un exemple simple de formule de Markush.

Comme mentionné plus haut, une formule de Markush est une représentation structurelle d'un composant ou d'une famille de composants chimiques. Elle se présente en général sous la forme d'un graphe ou d'un schéma comprenant une partie commune, ou groupe de base, (représenté par R₀ sur la figure 1), et une ou plusieurs parties de caractéristiques différentes, ou groupe "substituant", représentés sur la figure 1 par les symboles R₁ et R₂ et pouvant signifier l'un quelconque des atomes ou groupes d'atomes mentionnés à titre d'exemple sur la figure 1.

Compte tenu de ce type de représentation, on voit que le nombre de combinaisons, ou le nombre de graphes, issus d'une formule de Markush déterminée peut être très important. Ce nombre peut atteindre plusieurs millions de formules spécifiques différentes lorsqu'on utilise , par exemple, des termes génériques du type alkyle, etc.., pour désigner une ou plusieurs parties R₁ etc... du graphe de structure.

Sur la figure 2 on a représenté une formule de Markush telle qu'utilisée dans les représentations graphiques de saisie ou de sortie de données de la présente invention.

La formule, sur l'exemple représenté dans la figure 2, est constituée par un graphe de base, ou groupe de base, G₀. Ce graphe est souvent appelé graphe "père".

Il est connecté en deux de ces noeuds à deux graphes ou groupes "fils" G₁ et G₂. Les valeurs de G₁ sont représentées sur la figure comme pouvant être un atome d'hydrogène, de brome, de chlore ou d'iode; les valeurs prises par G₂ peuvent être, le brome, le chlore, l'iode ou deux sous-graphes ou groupes G₃, G₄, qui peuvent à leur tour prendre plusieurs valeurs différentes comme indiqué sur la figure 2.

Si chaque noeud représente habituellement un atome, dont l'identité peut être alors enregistrée, on a vu qu'un noeud pouvait également représenter un terme générique, comme alkyle, carboxyle, etc....

Dans la méthode et le système de l'invention, ces termes génériques sont avantageusement représentés par des "pseudo-atomes" appelés "super-atomes". Une table (table I) des atomes et super-atomes utilisables dans la méthode et le dispositif de l'invention est donnée en annexe, à titre d'exemple non limitatif.

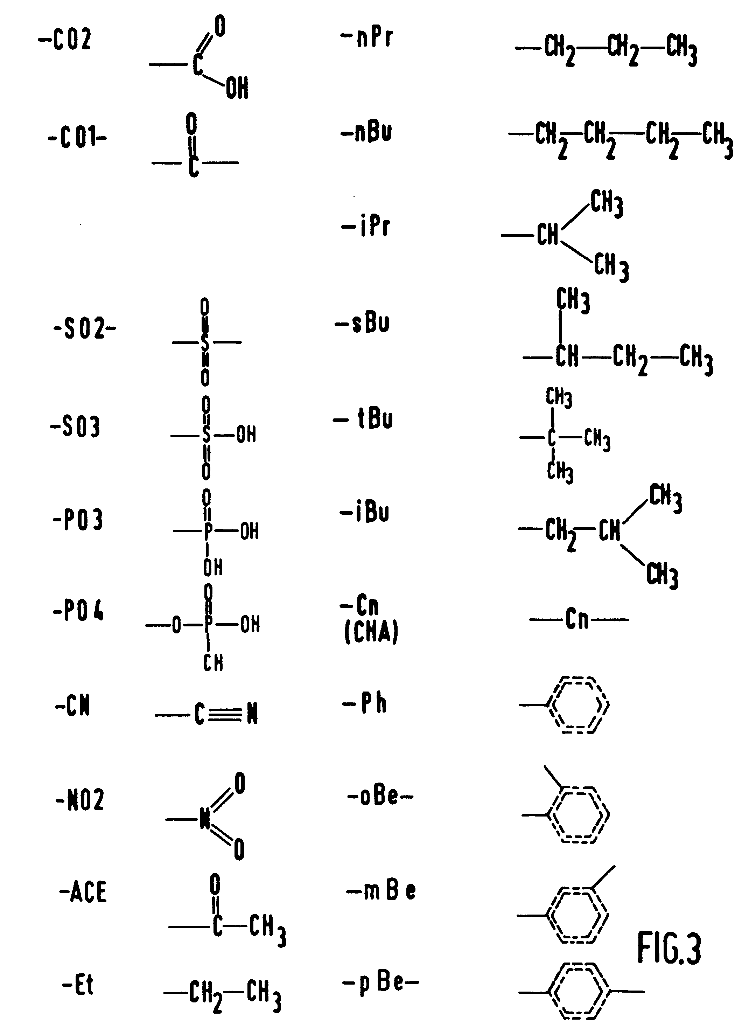

De plus, et dans un but de simplification, des ensembles d'atomes fréquemment rencontrés dans les formules chimiques de la littérature ont été définis. Ils permettent d'effectuer des recherches plus rapides, dites par "raccourcis". De tels exemples d'ensembles d'atomes, utilisables dans l'invention, sont représentés en figure 3.

Les "super-atomes" (table 1) ou les ensembles d'atomes (figure 3) sont utilisés dans la suite de l'invention comme n'importe quel autre atome.

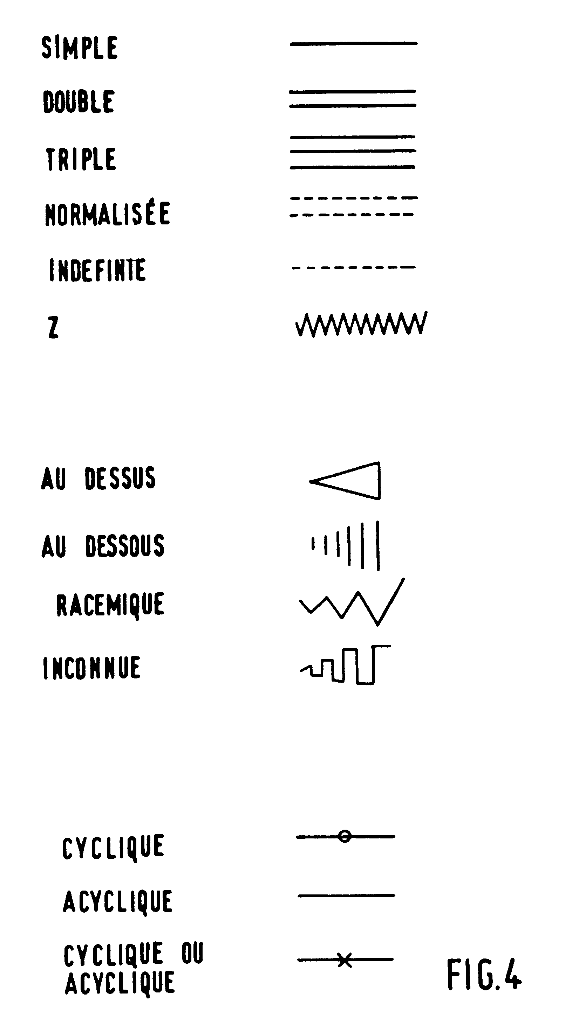

Les connexions entre noeuds d'une formule de Markush, qui représentent les liaisons, doivent également être définies, pour permettre une représentation suffisamment complète des molécules à stocker ou à rechercher. Les informations concernant ces liaisons, comme l'orientation ou la disposition relative dans l'espace des différents noeuds les uns par rapport aux autres (stéréochimie) sont mentionnées sur la figure 4 qui donne également un type de représentation graphique de ces liaisons, tel qu'avantageusement utilisées dans la présente invention.

Enfin, il s'avère utile de pouvoir préciser les spécifications des atomes des différents noeuds par d'autres informations que leur simple identité, par exemple leur charge, leur masse atomique, etc...

Pour ce faire, un mode de réalisation avantageux de stockage des formules selon l'invention prévoit de stocker les différents noeuds des graphes représentant la formule de Markush en affectant à chaque noeud une étiquette ou "attribut" pour faire état de ces informations.

On a représenté ci-après, et sans que cela soit en rien limitatif du nombre ou des types d'attributs envisageables, une liste d'attributs (table II) pouvant été affectés aux atomes ou groupes d'atomes des modes de représentation utilisés dans l'invention.

**TABLE II**

| NATURES D'ATTRIBUTS | | |
|---|---|---|
| 1 - | Charges | CH |
| 2 - | Isotopes | IS |
| 3 - | Sites Libres/Hydrogènes | SL |
| 4 - | Stéréochimie | ST |
| 5 - | Valences | VA |
| 6 - | Numérotation | NU |
| 7 - | Chaines carbonées/cycles | CC |
| 8 - | Multiplicateurs | MU |
| 9 - | Polymères/DL | PO |
| 10 - | Deutérium-Tritium | DT |
| 11 - | Renumérotation | RE |
| 12 - | Position | SP |

Il est également donné ci-après, à titre d'exemple, un mode de réalisation du codage de quelques uns de ces attributs sous forme numérique. (table III, IV, V et VI). Dans les tableaux ci-après (QU) signifie qu'il s'agit d'une information ne pouvant être fournie que dans le cadre d'une question.

**TABLE III**

| CHARGES | |
|---|---|
| 1 Mot numérique | |
| OCTET NO 1 : | |
| BIT 1 | Pas de charge (QU) |
| BIT 2 | Charge quelconque (QU) |
| BIT 3 | charge localisée |
| BIT 4 | charge délocalisée |
| BIT 5 | Charge positive |
| BIT 6 | charge négative |
| BIT 7 | |
| BIT 8 | |
| OCTET NO 2 : Valeur absolue de la charge | |
| L'absence d'attribut s'exprime par : CH = 0 | |
| Valeurs Min/Max : -9 à +9 | |

**TABLE IV**

| ISOTOPES | |
|---|---|
| | 1 Mot numérique |
| BIT 1 | Pas de masse (QU) |
| BIT 2 | Masse quelconque (QU) |
| BIT 3 | |
| BIT 4 | |
| BITS 5/16 | Valeur de l'isotope |
| L'absence d'attribut s'exprime par : IS = 0 | |
| Valeurs Min/Max : 2 à 1023 | |

**TABLE V**

| SITES LIBRES/HYDROGENES | |
|---|---|
| 1 Mot numérique | |
| SL = Nombre de sites Libres | |
| BIT 1 | (Pas de sites Libres) |
| BIT 2 | (Nombre de sites Libres quelconque) |
| BIT 3 | |
| BIT 4 | |
| BIT 5 | |
| BIT 6 | |
| BIT 7 | |
| BIT 8 | |
| BIT 9 | |
| BIT 10 | |
| BIT 11 | |
| BITS 12/16 | nombre de sites libres |
| L'absence d'attribut s'exprime par : SL = 0 | |
| Valeurs Min/Max : 0 à 16 | |

La notion de site libre est importante. Elle est utilisée pour spécifier la quantité ou le degré de substitution souhaité d'un atome ou d'une position. Si aucun site libre n'est spécifié, le système complète automatiquement en "équilibrant" la valence avec des atomes d'hydrogène. Le nombre de sites libres spécifié indique le nombre maximum de liaison(s) acceptable(s) pour un atome donné, en plus de celle(s) explicitement représentée(s) dans le graphe.

**TABLE VI**

| VALENCES | |
|---|---|
| | 1 Mot numérique |
| OCTET NO 1 : | |
| BIT 1 | Pas de valence (QU) |
| BIT 2 | Valence quelconque (QU) |
| BIT 3 | |
| BIT 4 | |
| BIT 5 | |
| BIT 6 | |
| BIT 7 | |
| BIT 8 | |
| OCTET NO 2 : | Valeur de la valence |
| L'absence d'attribut s'exprime par : VA = valence normale | |
| Valeurs Min/Max : 0 à 255 | |

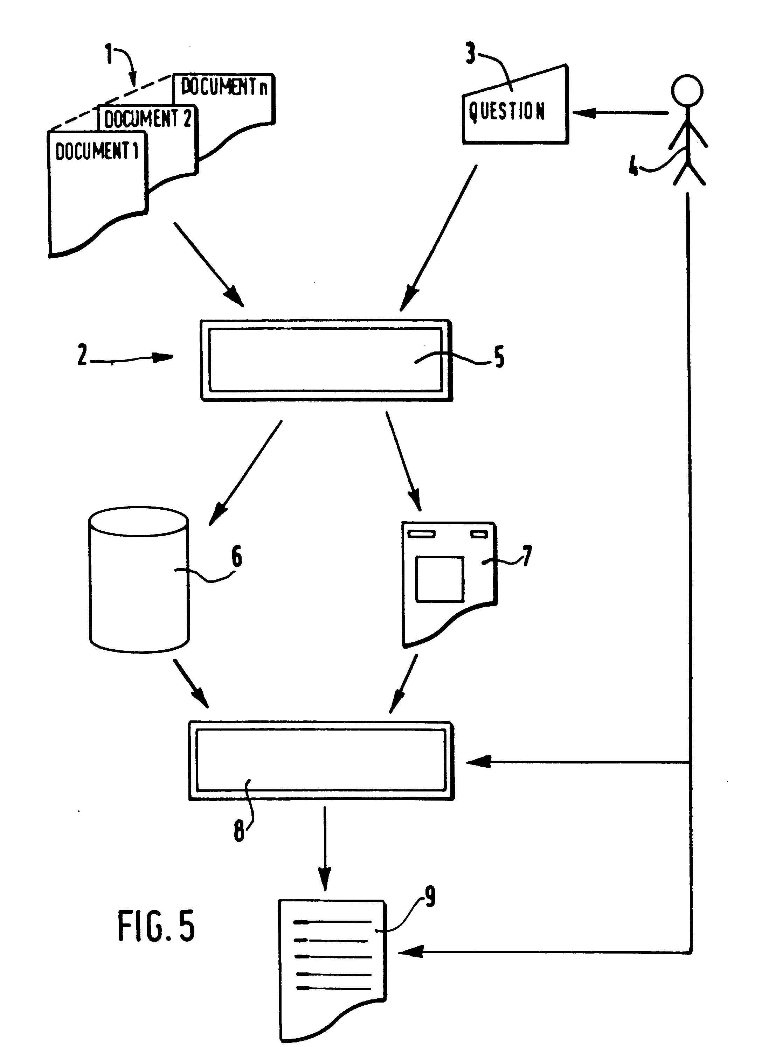

La figure 5 est un schéma de principe du système de stockage et de recherche de la formule de Markush selon le mode de réalisation de l'invention plus particulièrement décrit ici.

Ce mode de réalisation est avantageusement mis en oeuvre grâce à un ordinateur qui comprend, de façon connue en soi, une unité centrale (CPU) connectée à des dispositifs annexes comportant des unités de stockage externe et des moyens d'interfaces entrées - sorties, tels que des écrans de visualisation et des imprimantes, lesdits périphériques étant reliés à l'unité centrale de façon connue en soi par les moyens habituels de télécommunication.

L'unité centrale est par exemple constituée par un ou plusieurs ordinateurs IBM (INTERNATIONAL BUSINESS MACHINE) de grande capacité, par exemple du type IBM 3090. Les périphériques, ou terminaux, d'interfaces entrée-sortie peuvent être eux-mêmes des micro-ordinateurs du type IBM PC, ou d'autres terminaux du type Hewlett Packard 2647A, pour permettre l'entrée des données sous forme graphique et/ou alpha-numérique.

Sur la figure 5 on a représenté une série 1 de documents, (Document 1, Document 2..) comportant chacun une formule de Markush. Les formules de Markush de ces documents sont entrées dans le système 2 par l'intermédiaire d'un programme d'application, connu en lui même, permettant la représentation graphique et/ou alpha-numérique des formules, par exemple un programme du type PLOT 10 de la société TEKTRONIX., ou d'un autre type disponible sur le marché.

La formule de Markush recherchée, correspondant à la question 3, est saisie de façon sensiblement identique par l'opérateur 4.

Les formules de Markush issues des documents ou des questions sont ensuite traitées par le programme de saisie 5. Ce programme stocke les données constituées par les formules des documents entrées dans le système, dans un fichier 6 de base de données, sous forme de tables de connectivité comprenant chacune un numéro de référence de la formule et un numéro de présélection, comme cela va être décrit par la suite.

De la même façon la formule de Markush de la question 3 est formulée par le programme de saisie 5 sous forme de table de connectivité 7 avec un numéro de référence et un numéro de présélection. La formule recherchée 7 est ensuite comparée avec le fichier 6 de la base de donnée par un programme de recherche 8, sur le déroulement duquel l'opérateur 4 peut intervenir en interactif.

Le programme engendre une liste 9 après traitement, liste dont on extrait visuellement, et/ou de façon imprimée, les références et/ou représentation de la ou des éventuelles formules de Markush, issues du fichier 6 de la base de donnée, et qui correspondent à la formule de Markush recherchée 7.

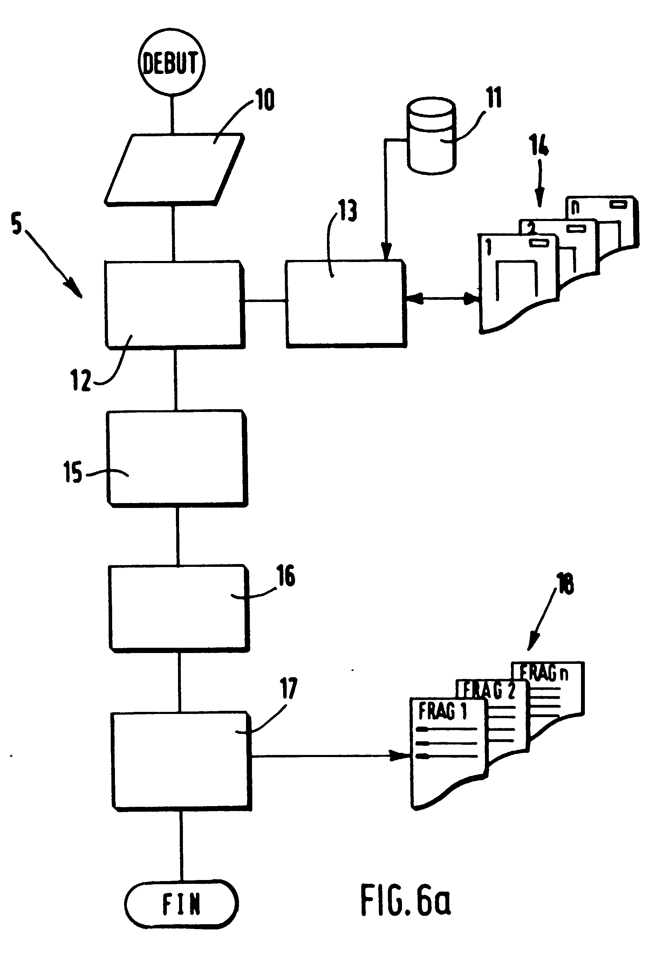

En se référant maintenant à la partie supérieure de la figure 6a, l'opérateur introduit dans le système sous forme graphique ou alpha-numérique, les formules de Markush de la banque de données et/ou de la formule à rechercher dans ladite banque de données (bloc d'introduction de données 10).

Cette introduction se fait comme on l'a mentionné plus haut, par l'intermédiaire d'un programme d'application connu en soi.

Par ailleurs, et de façon connue, les conventions et définitions nécessaires à la compréhension des formules de Markush saisies et traitées, ont été stockés au préalable en mémoire de l'ordinateur.

En particulier, les tables I à VI, et les conventions et définitions des figures 1 à 4, ont été introduites dans le système.

Un tableau VII représentant le tableau de numéros de présélection utilisés dans la suite du programme a été également constitué (référence 11 sur la figure 6a).

Un exemple de tableau VII est donné ci-après sous forme simplifiée pour mieux faire comprendre l'invention. L'exemple donné n'est, bien entendu, pas limitatif, notamment quand au nombre et au type de caractères types retenus. Avantageusement le nombre de caractères peut par exemple être de l'ordre de 100 à 400.

**TABLE VII**

| NO BIT | | DESIGNATION | |
|---|---|---|---|
| | | Description Formule Brute | |
| 1 | 8 Carbones | ou plus | |
| 2 | 12 Carbones | ou plus | |
| 3 | 16 Carbones | ou plus | |
| 4 | Pas d'Oxygène ou de Soufre | | |
| 5 | Pas d'Azote | | |
| 6 | 1 Oxygène | ou plus | |
| 7 | 2 Oxygènes | ou plus | |
| 8 | 1 Azote | ou plus | |
| 9 | 2 Azotes | ou plus | |
| 10 | 1 Halogène | ou plus | |
| 11 | 2 Halogènes | ou plus | |
| 12 | 1 Chlore | ou plus | |
| 13 | Pas d'autres Hétéro-atomes que : O, S, P, N, Hal | | |
| 14 | Pas d'Hétéro-atome | | |
| 15 | Pas d'Halogène | | |

| | | Description des Liaisons et Fonctions Chimiques | |
|---|---|---|---|
| Liaisons Doubles | | | |
| 16 | 1 Liaison | C = C | ou plus |
| 17 | 1 Liaison | C = N | ou plus |
| 18 | 2 Liaisons | C = N | ou plus |
| 19 | 1 Liaison | C = S | ou plus |

| Liaisons Triples | | | |
|---|---|---|---|
| 20 | 1 Liaison | C : C | ou plus |

| Fonctions Chimiques | | | |
|---|---|---|---|
| 21 | 1 fonction | O = N = O | ou plus |
| 22 | 1 fonction | C - O - C | ou plus |
| 23 | 1 fonction | O = C - S | ou O u C u S |
| | ou | O - C = S | ou plus |

| | | Description Noyaux Cycliques | |
|---|---|---|---|
| 24 | | Pas de noyau cyclique | |
| 25 | | 1 noyau cyclique ou plus | |
| 26 | | 2 noyaux cycliques ou plus | |
| 27 | | 1 monocycle aromatique | |
| 28 | | 1 noyau à 2 cycles exactement | |
| 29 | | 1 noyau à 3 cycles exactement | |
| 30 | | 1 noyau à 1 cycle ou plus | |

| | | Description des monocycles aromatiques | |
|---|---|---|---|
| 31 | | 1 monocycle sans Hétéroatome | |
| 32 | | 1 autre monocycle de même type | |

| | | Description des monocycles non aromatiques | |
|---|---|---|---|
| 33 | | 1 monocycle sans Hétéroatome | |
| 34 | | 1 autre monocycle de même type | |

| | | Description des noyaux à 2 cycles exactement | |
|---|---|---|---|
| 35 | | 1 noyau sans Hétéroatome | |
| 36 | | 1 autre noyau de même type (2 cycles exactement) sans Hétéroatome | |

| Description des noyaux à 3 cycles exactement | | | |
|---|---|---|---|
| 37 | | 1 noyau sans Hétéroatome | |
| 38 | | 1 noyau avec 1 Oxygène | ou plus |
| 39 | | 1 noyau avec 1 Liaison aromatique | ou plus |

| | | Description des noyaux à 1 cycle ou plus | |
|---|---|---|---|
| 40 | | 1 noyau avec Hétéroatome | |
| 41 | | 1 noyau avec 1 Azote ou plus | |
| | | Description des noyaux à 2 cycles ou plus | |
| 42 | 1 noyau avec Hétéroatome | | |
| 43 | 1 autre noyau de même type (2 cycles ou plus) avec Hétéroatome | | |
| 44 | 1 noyau avec 1 Liaison aromatique | | ou plus |
| 45 | 1 noyau avec 9 Liaisons aromatiques | | ou plus |

| | | Divers | |
|---|---|---|---|
| Attributs | | | |
| 46 | 1 Atome | portant une charge ou plus | |
| 47 | 1 Atome | isotope ou plus | |
| 48 | 1 Atome | portant de la stéreo | ou plus |
| 49 | 1 Atome | portant une valence | ou plus |
| 50 | 1 Atome | portant un ch. cyclique | ou plus |
| 51 | 1 Atome | portant un multiplicat. | ou plus |
| 52 | 1 Atome | portant une polymeris. | ou plus |

| | | Négations | |
|---|---|---|---|
| 53 | pas d'Hétérocycle | | |
| 54 | pas de Carbocycle | | |
| 55 | pas de cycle Insaturé | | |
| 56 | pas de cycle Saturé | | |
| etc... | | | |

Les formules de Markush sont saisies sous forme alphanumérique ou graphique comme indiqué précédemment. Elles comportent chacune un numéro externe attribué par l'opérateur (UID1) ainsi qu'un certain nombre d'informations complémentaires (date de saisie, texte associé à la formule de Markush tel qu'on veut le voir apparaître dans le fichier d'identification de la formule etc.). Les renseignements sur les différents noeuds de chaque formule saisie permettent la constitution des tables de connectivité.

Une table de connectivité est constituée par plusieurs tableaux. Un exemple de mode de réalisation de table de connectivité utilisée dans le procédé et le système selon l'invention est donné ci-après, (table VIII). La table comporte notamment avantageusement 5 premiers tableaux (DEG, ADR, TABGR, TABLI et TABST) définissant précisement chaque noeuds du graphe de la formule de Markush qu'elle représente, et 4 seconds tableaux (LIRAT, IFR, IAT et NVAL) définissant les relations entre les différents groupes comme on le verra plus précisemment dans la suite du texte.

**TABLE VIII**

| TABLE DE CONNECTIVITE | |
|---|---|
| - Description des informations générales | |
| | |
| - NOFM | numero interne attribue par le Logiciel numerique |
| - UID1 | numero externe attribue par l'utilisateur cadre a gauche |
| | alphanumerique 12 caracteres |
| - UID2 | numero interne attribue par le logiciel a |
| | partir d' informations de l'utilisateur numrique |
| - ANNEE | date de saisie attribuee par le logiciel |
| MMJJ | numerique |
| | ( ex : 1985 612 ---- > 12/06/85 ) |
| - LGTEX | longueur de TEXT en mots de longueur 4 numerique |
| TEXT | texte associe |
| | alphanumerique 256 caracteres |
| - BITMP | type de formule attribue par l'utilisateur |
| | numerique |
| - CNTRL | flags de controle |
| | numerique |
| - USER | userid |
| | alphanumerique 8 caracteres |
| - NCFB | longueur de FB en mots de longueur 4 |
| | numerique |
| FB | formule brute |
| | alphanumerique 256 caracteres |
| - NBFR | nombre de fragments |
| | numerique |
| IDEB | table des debuts de fragments |
| | numerique |
| IFRAC | table des coefficients de fragments |
| | numerique |
| ===> | IDEB (I) = J |
| | IFRAC (1, I) = K1 |
| | IFRAC (2, I) = K2 |
| | où I = numero du fragment |
| | J = numero d'atomes du debut du fragment |
| | K1 = coefficient du fragment ( numerateur ) |
| | K2 = coefficient du fragment ( denominateur ) |
| ===> | IDEB(I+1)-1 est le numero du dernier atome du |
| | fragment I |
| - NBGM | plus grand numero de groupe defini |
| | numerique |
| - NBGR | nombre de groupes definis |
| | numerique |
| - NOGA | table de positionnement des groupes |
| | numerique |
| ===> | NOGA (I) = J |
| | I = numero du groupe |
| | J = 1024 * numero du groupe pere + numero de l'atome G |
| - NBLP | table du nombre de liaisons de rattachement des groupes |
| | numerique |
| ===> | NBLP (I) = J |
| | I = numero du groupe |
| | J = nombre de liaisons de rattach. du groupe |
| | -1 pour un groupe dont les liaisons de rattachement ne sont pas definies |
| | -2 Pour un groupe non existant |
| - LIRAT | table des atomes de rattachement des groupes |
| | numerique |
| ===> | LIRAT (I,K)= J |
| | I = numero du groupe |
| | K = numero de la liaison de rattachement |
| | J = numero de l'atome de rattachement voisin dans le groupe pere |
| | - numero de la liaison de rattachement pour un graphe de graphe porteur de liaison de rattachement |
| - NVAL | table du nombre de valeurs des groupes |
| | numerique |
| ===> | NVAL (I) = J |
| | I = numero du groupe |
| | J = nombre de valeurs du groupe |
| - NHYD | table des groupes a valeur Hydrogene |
| | numerique |
| ===> | NHYD (I) = J |
| | I = numero du groupe |
| | J = 0 groupe non Hydrogene |
| | 1 groupe Hydrogene |
| - NUGEX | table de numerotation externe des groupes |
| | numerique |
| ===> | NUGEX (I) = J |
| | I = numero du groupe |
| | J = numero externe du groupe |
| - IFR | table d'adresse par groupe vers IAT |
| | numerique |
| LGIAT | longueur de IAT en mots de longueur 2 |
| | numerique |
| IAT | table des debuts de valeurs des groupes |
| | numerique |
| ===> | IFR (I) = J |
| | IAT (J) = K |
| | I = numero du groupe |
| | J = adresse vers IAT correspondant a la 1ere valeur du groupe |
| | K = numero du fer atome de la valeur |
| ===> | pour connaitre le debut de chaque valeur du groupe I |
| | IAT ( IFR(I) ) a IAT ( IFR(I+1)-1 ) |
| - NBL | nombre de listes d' attachement indetermine |
| | numerique |
| ADRAD | table d' adresse vers LISAD |
| | numerique |
| LISAD | table des listes d'atomes impliques dans un attachement indetermine |
| | numerique |
| ===> | ADRAD (I) = J |
| | LISAD (J) = K |
| | I = numero de l'attachement indetermine |
| | J = adresse vers LISAD correspondant a la description de la liste d'atomes de l' attachement indetermine |
| | K = 1er numero d'atome de la liste |
| ===> | pour avoir la liste des atomes impliques dans l'attachement indetermine I : |
| | LISAD ( ADRAD(I) ) a LISAD ( ADRAD(I+1)-1 ) |
| - LGBS | longueur de BS en mots de longueur 2 |
| | numerique |
| BS | informations de type bit-screen associees |
| | numerique |
| - NCBS | nombre de fragments pour la recherche bit-screen |
| | numerique |
| - MPBIT | BITMP ( OR,AND,NOT) pour la recherche bit-screen |
| | numerique |
| - LIMBS | limites diverses pour la recherche bit-screen |
| | numerique |

| - Description d'un graphe de formule | |
|---|---|
| | |
| - NAT | nombre d' atomes |
| | numerique |
| - DEG | table des degres des atomes |
| | numerique |
| ===> | DEG (I) = J |
| | I = numero de l'atome |
| - ADR LG | J = nombre de voisins de l'atome dans certains cas ( search AA ) , cette valeur est decrementee du nombre de voisins a valeur hydrogene pour faciliter le traitement table d'adresse vers TABGR , TABLI , TABST |
| | numerique longueur des tables TABGR , TABLI , TABST numerique |
| TABGR | table des numeros de voisins |
| | numerique |
| TABLI | table des natures de liaisons |
| | numerique |
| TABGR | table de stereochimie |
| | numerique |
| ===> | ADR (I) = J |
| | TABGR (J) = K1 |
| | TABLI (J) = K2 |
| | TABST (J) = K3 |
| | I = numero de l'atome |
| | J = adresse vers TABGR , TABLI , TABST |
| | K1= numero du fer voisin de I |
| | K2= nature de la liaison I - K1 |
| | K3= stereochimie de la liaison I - K1 |
| ===> | pour avoir la liste des voisins de l'atome I , ainsi que les natures de liaisons les joignant : |
| | TABGR ( ADR(I) ) a TABGR ( ADR(I+1)-1 ) |
| | TABLI ( ADR(I) ) a TABLI ( ADR(I+1)-1 ) |
| | TABST ( ADR(I) ) a TABST ( ADR(I+1)-1 ) |
| - AT | table des natures d'atomes |
| | numerique |
| ===> | AT (I) = J |
| | I = numero de l'atome |
| | J = nature de l'atome ( voir Annexe 2 ) |

Une fois, les informations concernant les formules de Markush introduites dans le système à l'aide du programme de saisie graphique et/ou alphanumérique mentionné ci-dessus, les tables de connectivité créées, sont traitées par le programme "Mise en forme du fichier" représenté par le bloc fonctionnel 12 sur la figure 6a.

Avec ce programme, on attribue à chaque formule de Markush un numéro d'identification (NOFMi) et un numéro de présélection (Npsi) décrivant synthétiquement des caractères types prédéterminés de la formule, en référence à la table constituée préalablement desdits caractères types (table VII).

Le numéro de présélection est avantageusement calculé automatiquement. Ce calcul est représenté schématiquement par le bloc fonctionnel 13 sur la figure 6a.

De façon plus précise, dans le mode réalisation de l'invention plus particulièrement décrit ici, le numéro de présélection, est calculé pour se présenter sous la forme d'une chaine de n bits par exemple de 200 bits, dont chaque bit correspond à un critère de sélection fixe. La valeur 1 correspond à la présence de ce critère, la valeur 0 ne correspondant pas forcément à l'absence de ce critère. Le calcul s'effectue à partir des tables de connectivité introduites, par l'intermédiaire d'un algorithme (GENBIT) dont un exemple de représentation en pseudo-code, compréhensible pour l'homme du métier, est fourni ci-après (table IX).

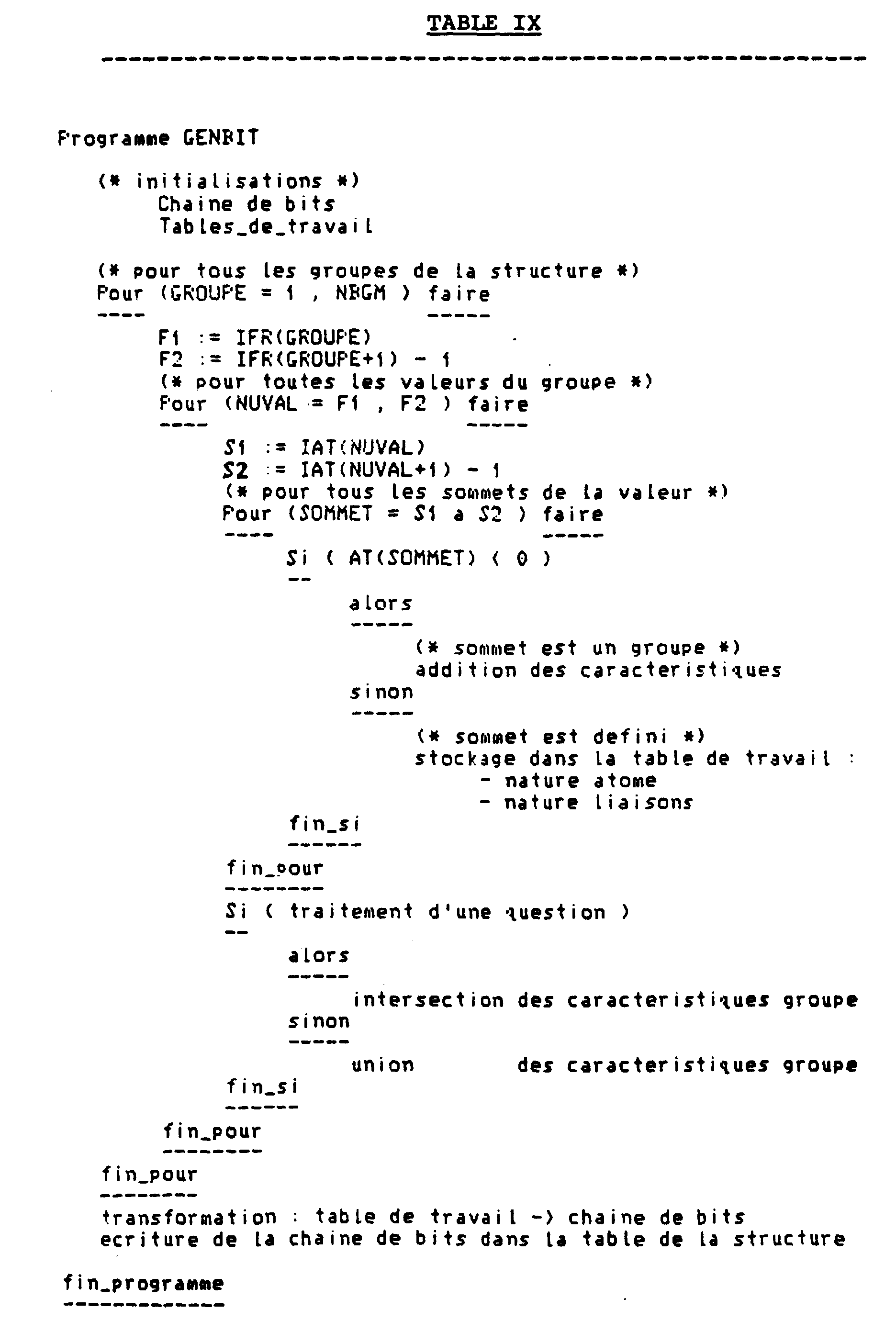

Le fichier de base 14 de formules chimiques obtenu après ce premier traitement, se présente comme une série de fiches par formule, référencées NOFM₁, NOFM₂, ... NOFMₙ, comprenant chacune une table de connectivité correspondante TC₁, TC₂ .... TCₙ et un numéro de présélection Nps₁, Nps₂ ... Npsₙ.

A partir de ces fiches, il est déterminé pour chaque formule traitée la liste de tous les noeuds, où atomes foyers, comprenant au moins deux voisins immédiats. Cette étape est représentée sur la figure 6a par le bloc fonctionnel 15.

On calcule ensuite (bloc fonctionnel 16) pour chaque noeud de cette liste, toutes les parties possibles où "fragments" de ladite formule issues de ce noeud, un fragment étant défini comme une partie de structure de formule constituée par un foyer, ses atomes voisins immédiats (environnement 1) et s'ils existent le ou les atomes voisins immédiats (environnement 2) de chacun des atomes voisins immédiats du foyer.

Enfin, on forme (bloc 17) un fichier de fragments 18 comprenant pour chaque fragment calculé des formules du fichier de base 14, la liste des numéros de référence des formules comprenant ce fragment ( FRAG₁ , FRAG₂......).

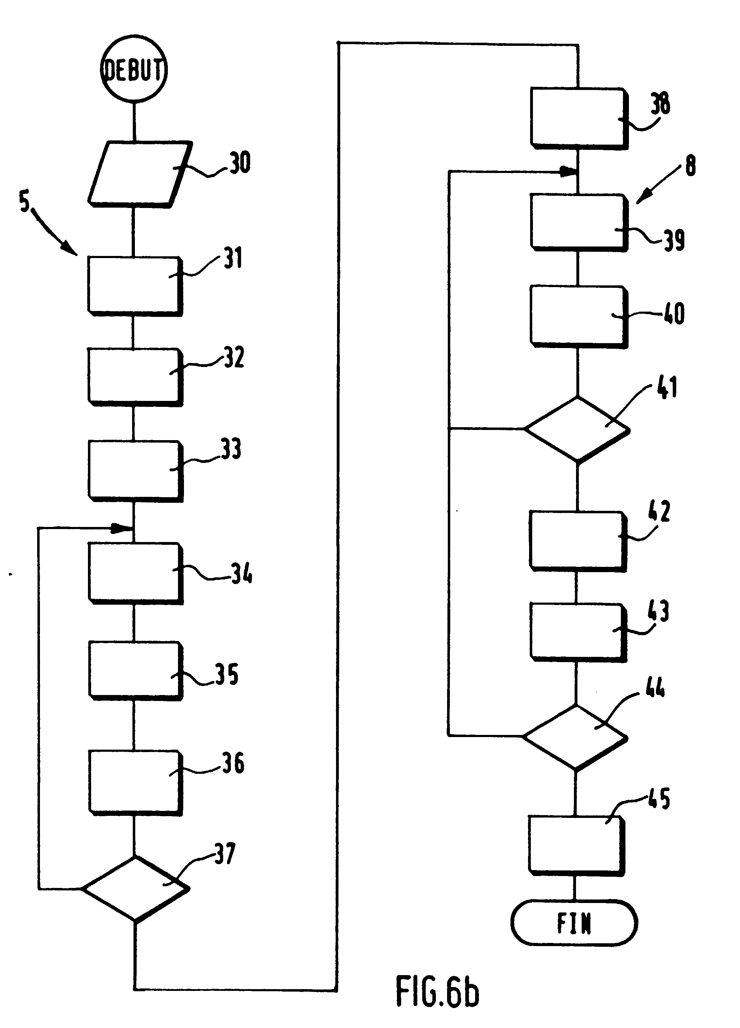

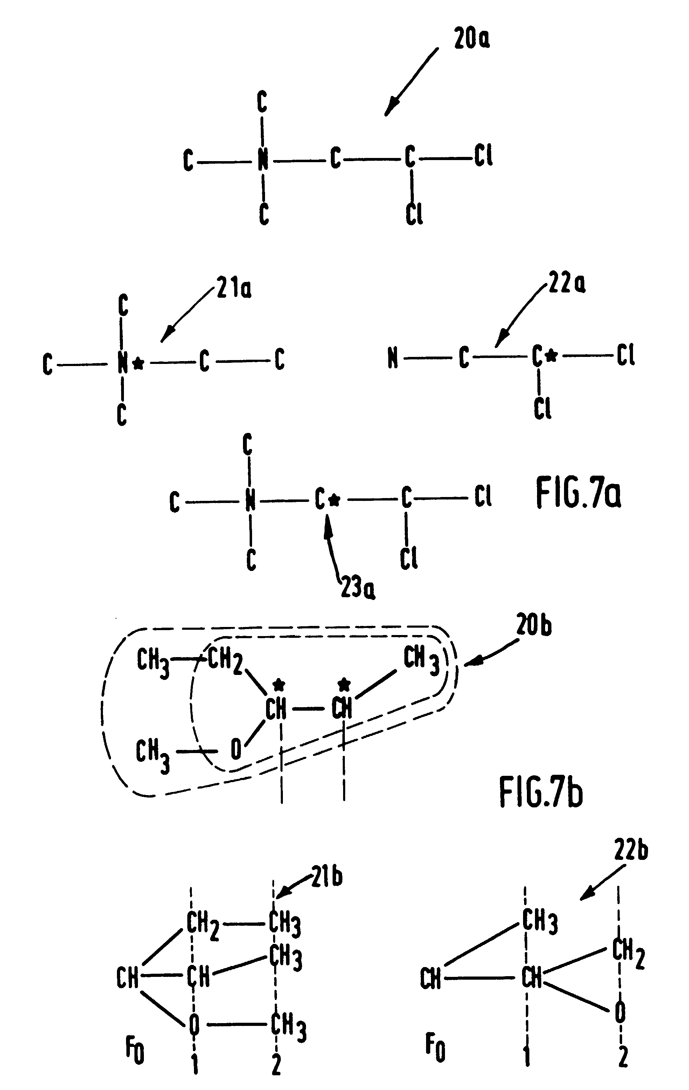

Avant de décrire la figure 6b et pour mieux faire comprendre la notion de "fragment à deux environnements", deux exemples de formules et de leurs fragments à deux environnements ont été représentés sur les figures 7a et 7b. La figure 7a montre une structure 20a. Elle comporte trois fragments à deux environnements 21a, 22a et 23a, dont les foyers sont repérés par un astérisque.

La figure 7b montre une autre structure 20b. Elle comporte deux fragments à deux environnements 21b et 22b, avec une présentation différente de celle de la figure 7a, les foyers étant toujours repérés par des astérisques.

Un fragment à deux environnements peut également être défini comme une sous structure bâtie concentriquement autour d'un foyer, le foyer étant le sommet de ladite sous-structure et comportant au minimum deux atomes voisins déterminés, chaque voisin étant relié au foyer par une liaison déterminée (ces deux conditions définissant l'environnement 1 du foyer), ladite sous-structure s'étendant à tous les sommets ou atomes voisins des atomes voisins de l'environnement 1, c'est-à-dire ayant une distance de "2" par rapport au foyer du fragment. Cette deuxième rangée d'atomes forme l'environnement 2. On se référera également aux traits interrompus de la figure 7b.

On fournit ci-après en pseudo-code (tables XI, XII, XIII et XIV) les étapes principales du programme, mettant en oeuvre les fragments, de la figure 6a de l'invention. Un premier tableau (table X) explicite les abréviations utilisées, le principe de présentation des fichiers de fragments à deux environnements (désignés par "frags" dans les tables) étant exposé ci-après.

### Principe de description des fragments

Trois fichiers décrivent avantageusement les fragments à deux environnements. Ces fichiers sont reliés selon le schéma suivant :
FRAG 1 ----> FRAG 2 ----> FRAG N

FRAG 1 est le fichier de "sous fragments". Il contient la description des "sous fragments" à un environnement (1) de la base de données. A chaque sous fragment 1 est associée une liste de positions 2 possibles. Cette liste est décrite dans FRAG 2.

Un ou plusieurs blocs directeurs, situés en tête du fichier, permettent de limiter la recherche sur les listes 2 associées à un sous fragment 1.

FRAG 2 est le fichier des listes 2. Il contient la description des listes de positions 2 associées à chaque sous fragment 1. A chaque jeu de positions 2 correspond une liste de numéros de structures décrite dans FRAG N.

FRAG N est le fichier de listes de numéros de structures ou de formules. Il contient la description des listes de numéros de structures associées à un Fragment à deux environnements.

Le lien entre ces trois fichiers se fait par adressage direct.

**TABLE X**

| ABBREVIATIONS | |
|---|---|
| Fichiers | |
| - TBLMAJ | fichier de travail (tables de connectivite structures) |
| - ELINCO | fichier de travail (liste des structures eliminees) |
| - FRAG0TRI | fichier de travail (fragments tries) |
| - FRAG0NCO | fichier de travail (fragments generes) |
| - FRAG2NCO | fichier de travail (fragments generes : 2 voisins) |
| - FRAG3NCO | fichier de travail (fragments generes : 3 voisins) |
| - FRAG4NCO | fichier de travail (fragments generes : 4 voisins) |
| - FRAG5NCO | fichier de travail (fragments generes : > 4 voisins) |
| | |
| Donnees | |
| - BUFF1 | buffer recevant fragment courant |
| - BUFF2 | buffer recevant fragment lu |
| - OCC | occurrence du fragment courant |
| - MAXOCC | occurrence maximum autorisee pour un fragment |
| | |
| Programmes | |
| - COMPV | comparaison de 2 chaines ( resultat : < , =, > ) |

Le programme décrit dans la table XI, détermine pour chaque formule de Markush entrée dans le système, la liste de tous les foyers de fragments. Pour chaque fragment ou "frag", il calcule tous les fragments possibles issus de ce foyer.

Enfin il élimine les formules générant trop de fragments et écrit dans le ficher ELINCO le numéro NOFMᵢ de ces formules.

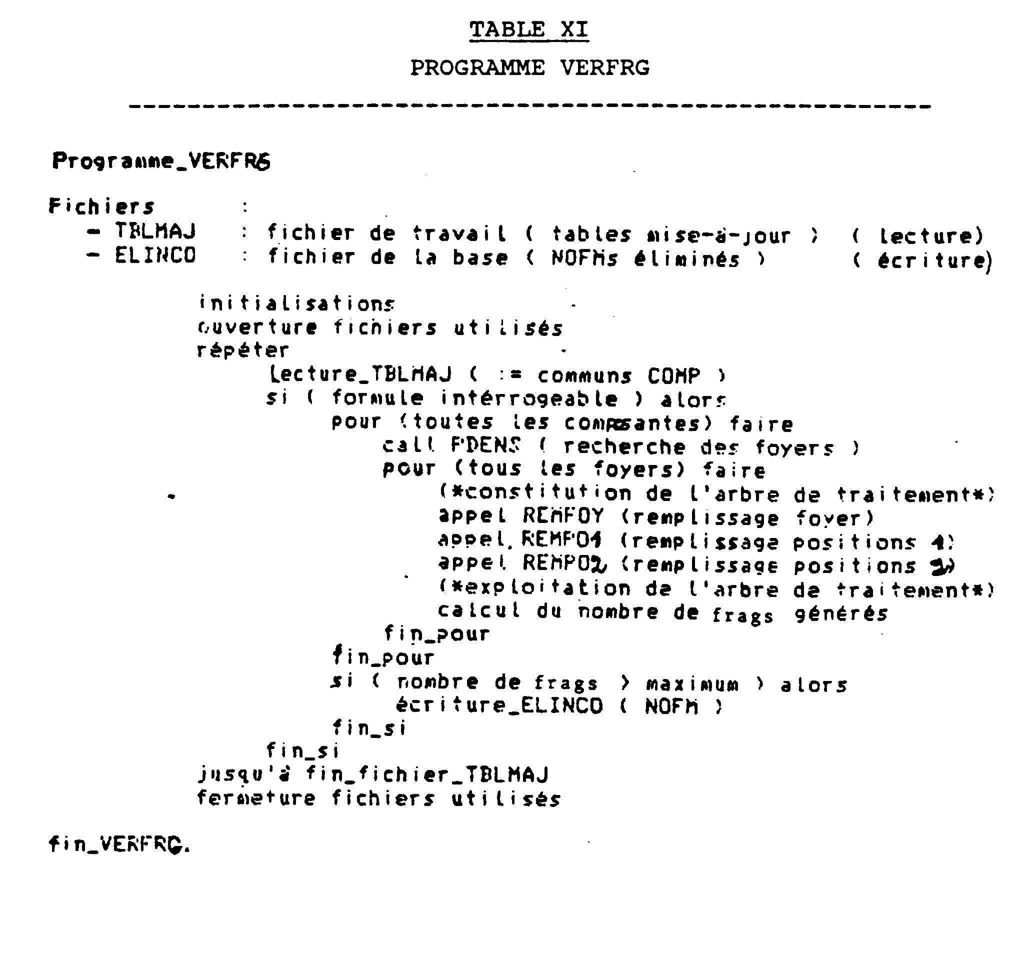

La table XIbis donne, à titre d'exemple, des précisions sur les procédures en pseudocode, mises en oeuvre dans des modes de réalisation avantageux de l'invention. Elles permettent, notamment, grâce à l'utilisation des tables de connectivité à tiroir et leurs tableaux LIRAT ou NVAL, les gains de temps important dans le traitement des données. Au lieu de recalculer tous les éléments nécessaires, à chaque fois, l'existence de tels tableaux permet d'aller y chercher directement, comme dans un "tiroir", l'information recherchée pour la suite des opérations de calcul.

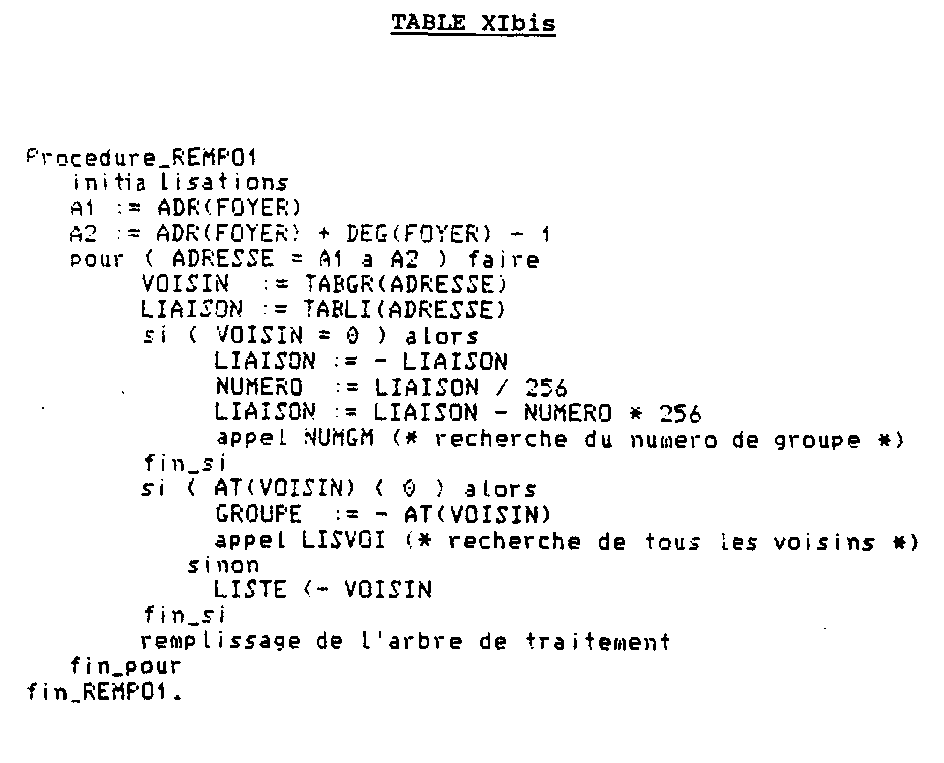

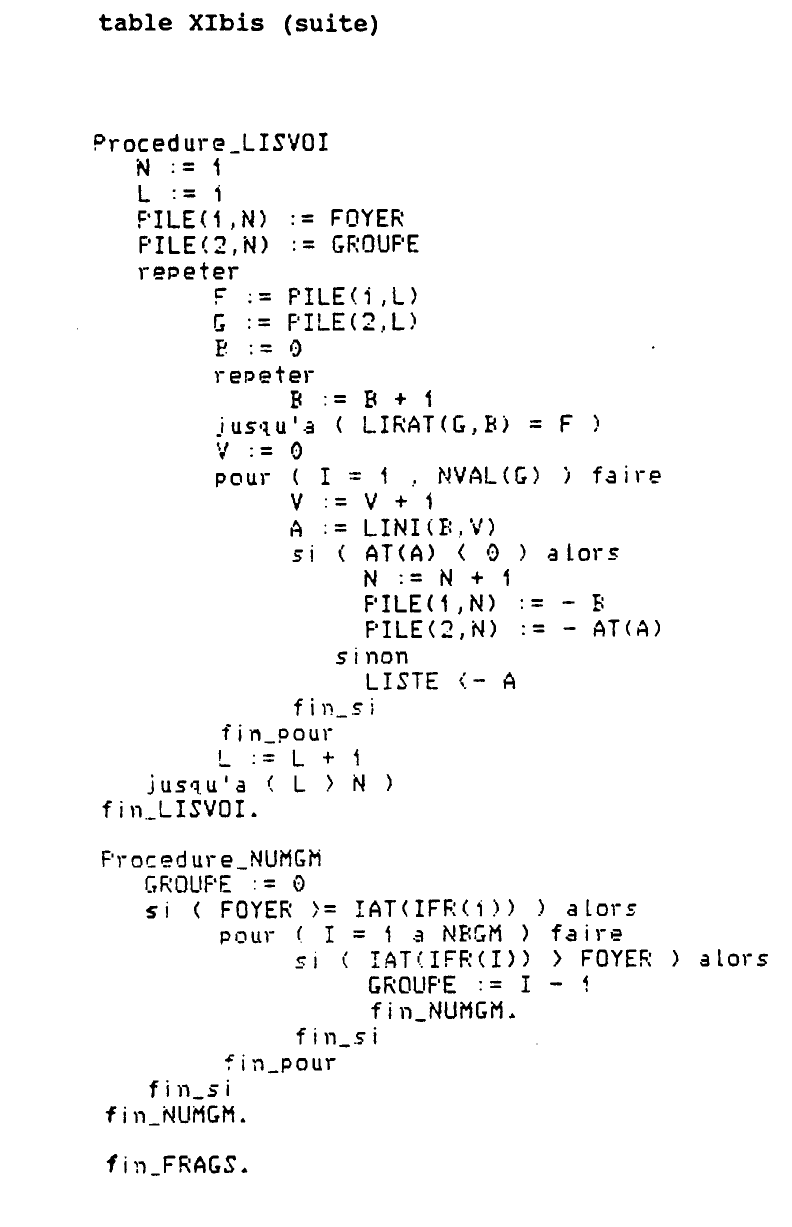

Le programme en pseudo-code de la table XII détermine pour chaque formule de la mise à jour la liste de tous les foyers de "fragments" à deux environnements.

Pour chaque foyer, il calcule tous les fragments possibles issus de ce foyer. Pour chaque foyer, il tente de ne retenir que les fragments significatifs, c'est à dire d'éliminer la redondance de fragments (calcul d'une clé de Hash coding sur le fragment). Puis il écrit pour tous les foyers, les fragments ainsi retenus dans le ficher FRAGONCO. Il édite également les statistiques de la génération, c'est à dire notamment le nombre de fragments calculés pour chaque formule.

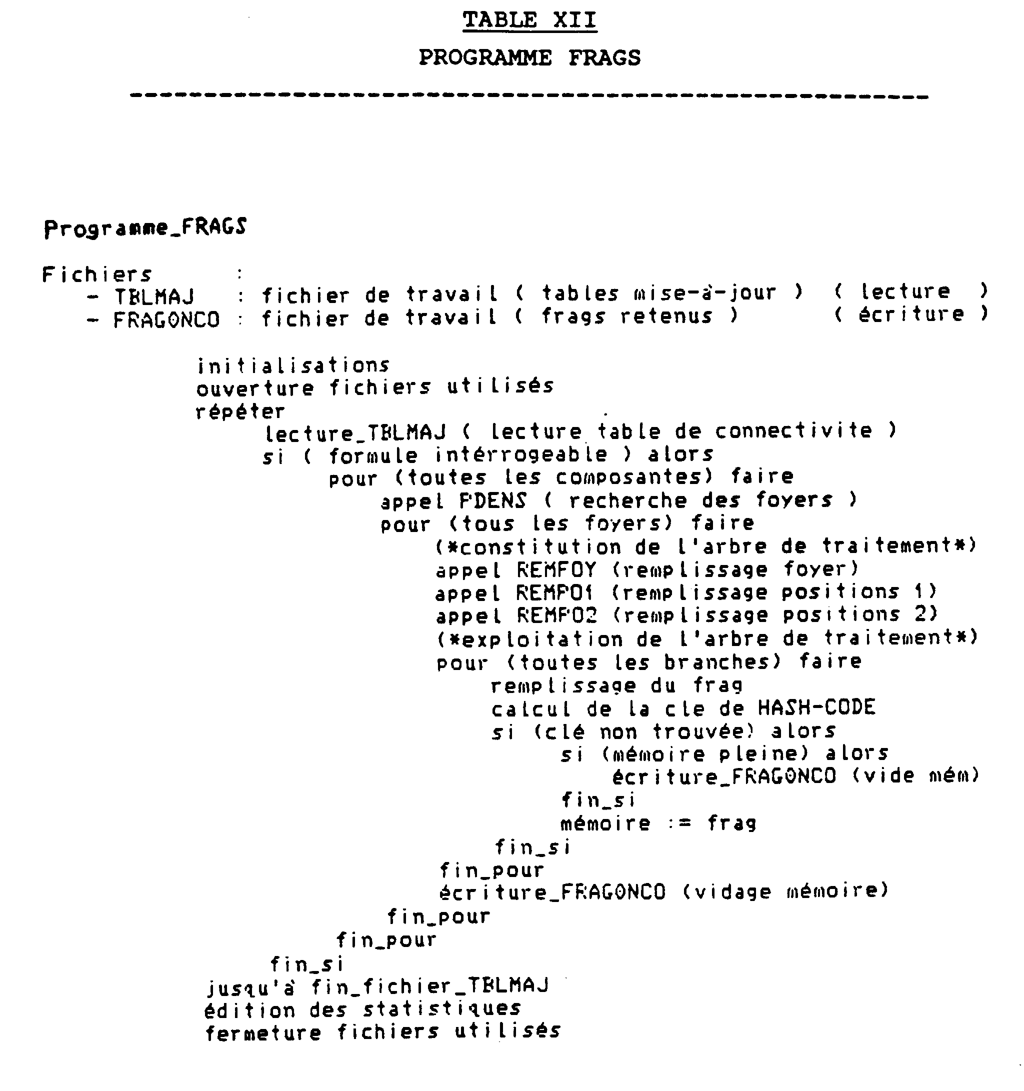

Après le tri de tous les fragments générés pour tous les foyers de toutes les formules de la table, le programme, décrit dans la table XIII, détecte la redondance entre les "fragments" et élimine les fragments identiques d'un même foyer.

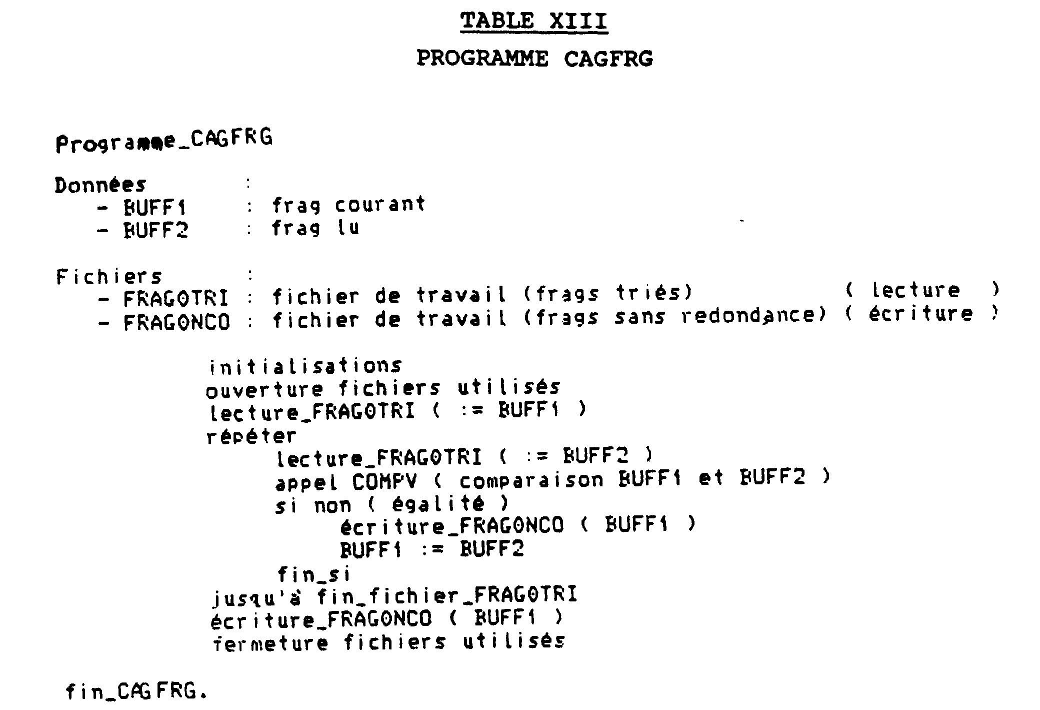

Enfin après le tri des fragments retenus, le programme (table XIV) détecte la redondance inter-fragment et calcule l'occurrence de chaque fragment à l'intérieur de chaque formule introduite (mise à jour).

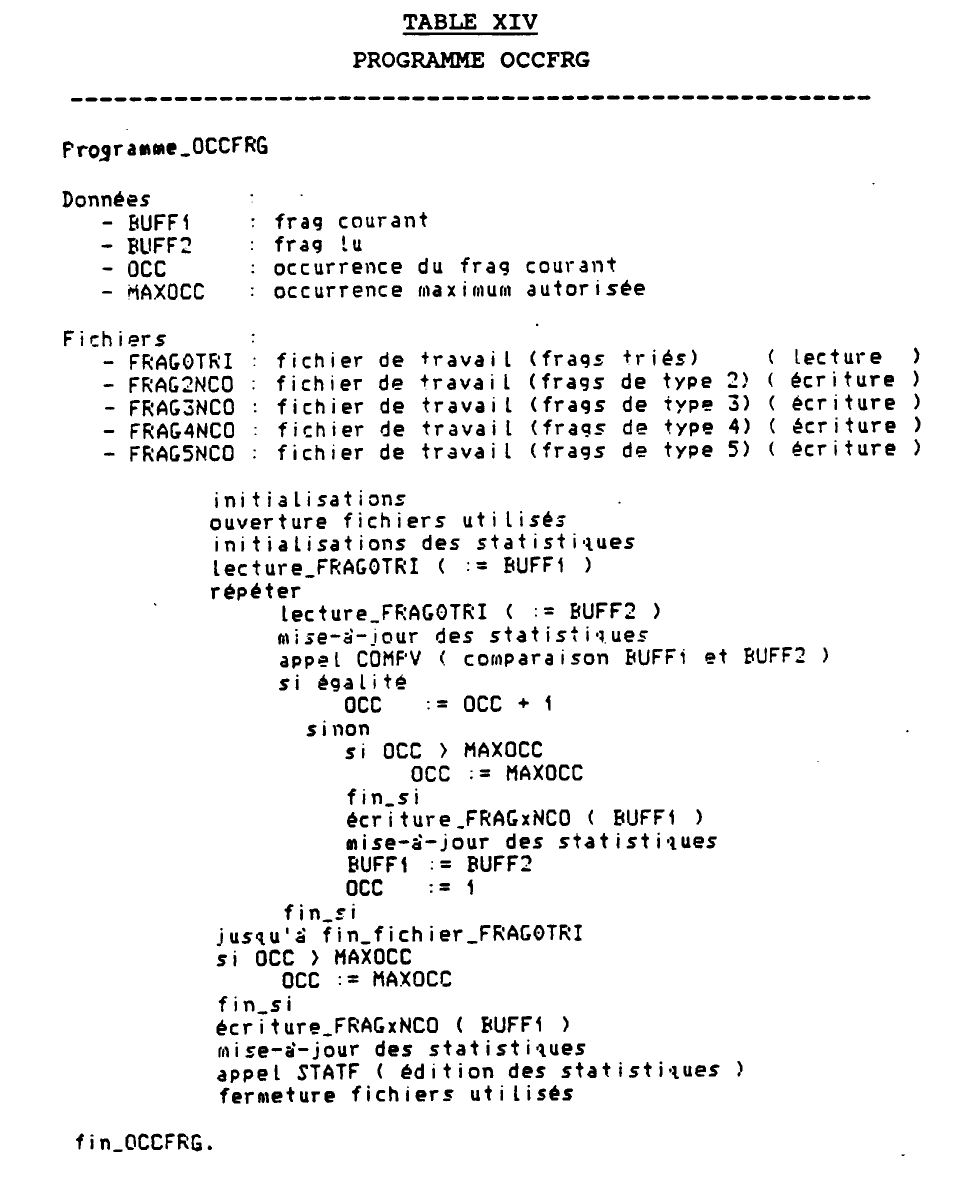

La figure 6b est un organigramme simplifié du programme de recherche d'une formule de Markush selon le mode de réalisation de l'invention plus particulièrement décrit ici.

En commençant par le haut de la figure 6b, la formule de Markush recherchée est introduite (bloc d'introduction de données 30), comme explicité précédemment.

La liste de ses noeuds, ou foyers, comprenant au moins deux voisins immédiats à l'origine d'un fragment étant défini comme une partie de structure de formule constituée par un foyer, ses atomes voisins immédiats (environnement 1) et s'ils existent le ou les atomes voisins immédiats (environnement 2) de chacun des atomes voisins immédiats du foyer, est ensuite déterminée (bloc 31).

On calcule (bloc 32) pour chaque noeud, tous les fragments possibles issus de ce noeud et on constitue (bloc 33) la liste de ces fragments pour la formule recherchée.

Pour un premier "fragment" de la formule de Markush recherchée, fragment avantageusement choisi comme étant le fragment commun à la formule recherchée et à un nombre minimum de formules du fichier de fragments 18 avec lequels on la compare (bloc 34), on extrait (bloc 35) du dit fichier 18 une première liste du numéro de référence de formules.

On compare ensuite (bloc 36), cette première liste avec la liste obtenue pour le fragment précédent, s'il y a lieu.

Dans le cas du premier fragment cette liste obtenue pour le "fragment précedent" ne comporte bien évidement pas de numéro.

De façon automatique, ou commandé manuellement par l'opérateur, l'opération peut être renouvelée (bloc de décision 37) pour le fragment suivant, et ainsi de suite.

Autrement dit, on compare (34) et on extrait (35) du fichier 18 une deuxième liste de numéro de référence de formules possibles comprenant un deuxième fragment. Cette liste est comparée (36) à la première liste obtenue pour le premier fragment, pour former une troisième liste "issue de fragment", constituée par les numéros communs des deux premières listes, et on réitère l'opération tant qu'on l'estime nécessaire (37), en comparant chaque nouvelle liste de numéros de formules possibles correspondantes à un nouveau fragment avec la liste constituée par les numéros communs de toutes les listes précédentes, pour former une liste de fragments "de longueur optimisée", que l'on lit (bloc 38).

On effectue ensuite une recherche sur numéro de "présélection" dont on a vu précédemment comment il a été constitué.

La recherche sur le numéro de "présélection" constitue un crible, qui va permettre de minimiser encore le nombre de candidats, en retardant ainsi les décompactages des tables de connectivité des candidats et les comparaisons noeud à noeud, très lourdes en temps d'utilisation de l'unité centrale (CPU), de ces formules avec la formule recherchée.

Ceci a pour effet d'étendre virtuellement la capacité de l'unité centrale et permet d'accélerer notablement la recherche.

Rappelons que le numéro de présélection se présente, comme on l'a vu, sous la forme d'une chaine de n bits, dont chaque bit correspond à un critère de sélection fixe. La valeur 1 correspond par exemple à la présence du caractère, la valeur 0 ne correspondant pas, du moins au niveau de la recherche de la formule de Markush recherchée, à l'absence de caractère.

La comparaison du numéro de présélection de la formule recherchée à celui du ou des candidats, se fait en recherchant l'inclusion logique stricte du deuxième dans le premier (ET logique, et égalité du résultat avec le numéro de présélection de la formule). Une liste de critères de sélection est donnée à titre d'exemple dans le table VII, comme on l'a vu.

Les étapes suivant l'étape 38 de lecture de la liste "issue de fragments de longueur optimisée", sont essentiellement: lecture du numéro de présélection de la formule recherchée (39), comparaison des numéros de présélection (40) de la formule recherchée avec ceux de la liste issue de fragments de longueur optimisée et, une fois la comparaison terminée (bloc de décision 41), poursuite du programme.

Dans le cas contraire, on réitère le recherche du numéro de présélection.

Une fois cette recherche de présélection effectuée, il y a lecture du fichier réponse et decompactage (bloc d'opération 42) des tables de conectivités des formules retenues.

Une liste finale formée par les numéros de référence des formules ayant un numéro de présélection identique à celui de la formule de Markush recherchée, peut ainsi être exploitée.

Dans un mode de réalisation préféré de l'invention, on effectue de plus, une comparaison noeud à noeud des formules de la liste finale, avec la formule de Markush recherchée (bloc 43). Cette comparaison est par exemple effectuée grâce à un algorithme tel que celui figurant dans le tableau XV ci-après.

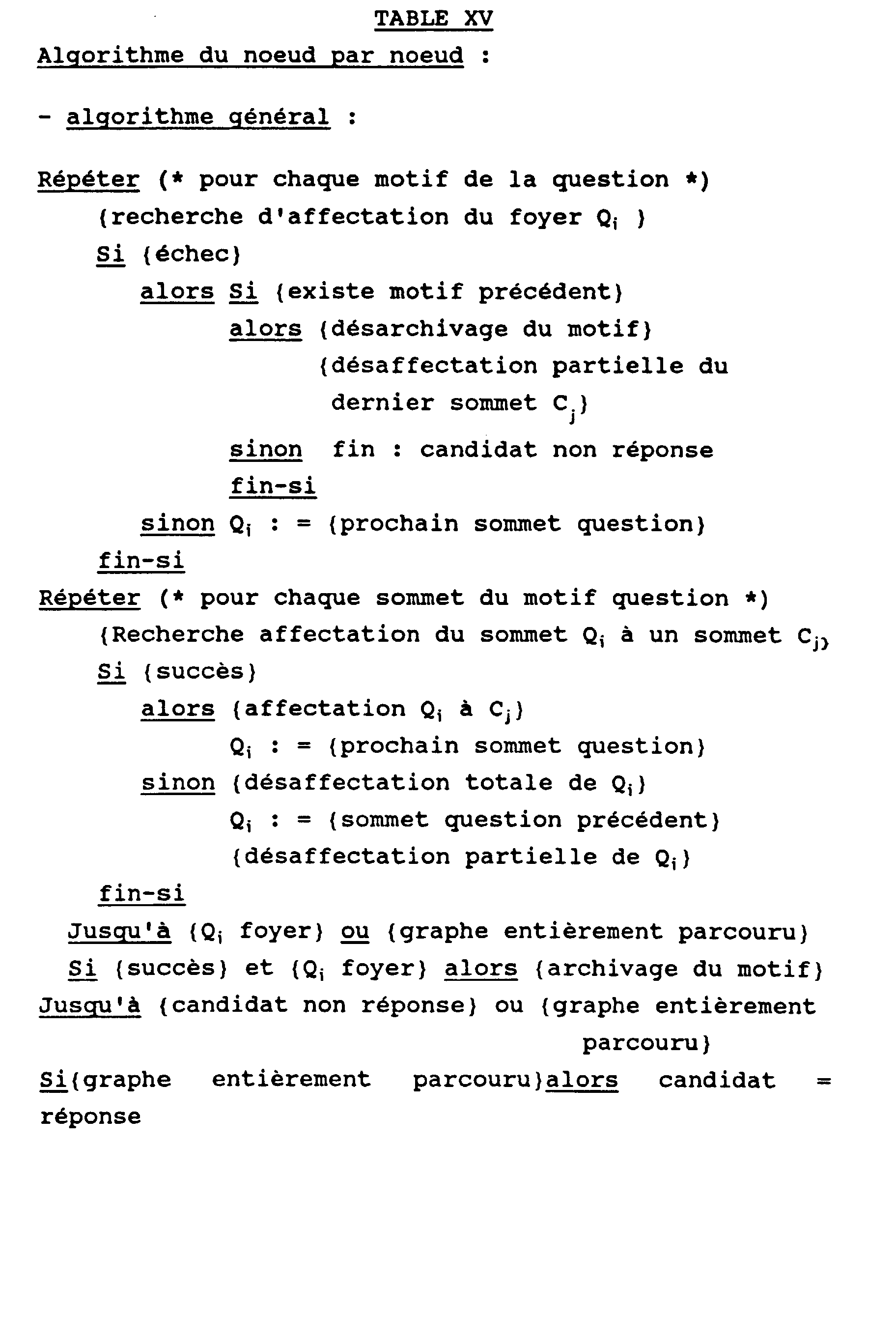

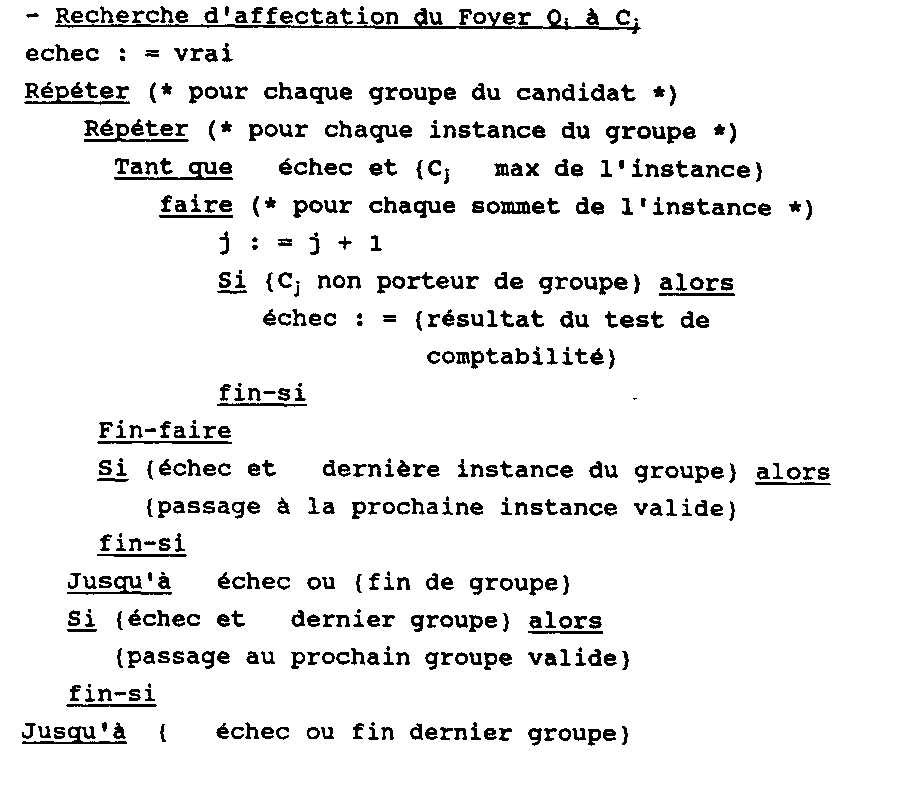

L'opération est renouvelée, par exemple par commande manuelle de l'opérateur, jusqu'à optention de résultats satisfaisants ou d'absence de résultat (bloc de décision 44).

Avantageusement, dans le cas où cette comparaison serait trop longue, il peut être prévu des circuits de "délestage" automatique, permettant seulement exceptionnellement une comparaison noeud à noeud, plus longue.

Une fois cette comparaison effectuée, on extrait les références (bloc 45) des formules de Markush éventuellement obtenues.

Avantageusement, bien que cela ne soit pas représenté sur l'organigramme, d'autres critères de sélection permettant d'affiner encore la liste des candidats, sont utilisables et utilisées dans des modes de réalisations préférés de l'invention.

Il s'agit notamment, et comme on l'a vu, de l'utilisation des attributs comme critère supplémentaire de sélection.

On ne recherche alors dans les fichiers de formules de Markush que les formules comportant une structure munie du ou des attributs spécifiquement affectés aux noeuds de la formule recherchée. Cette recherche basée sur des entrées de sélection supplémentaire s'effectue de façon connue en soi.

Pour illustrer l'invention, on donne ci-après un exemple de formule de Markush, telle que présentée pour être mise en oeuvre dans ladite invention.

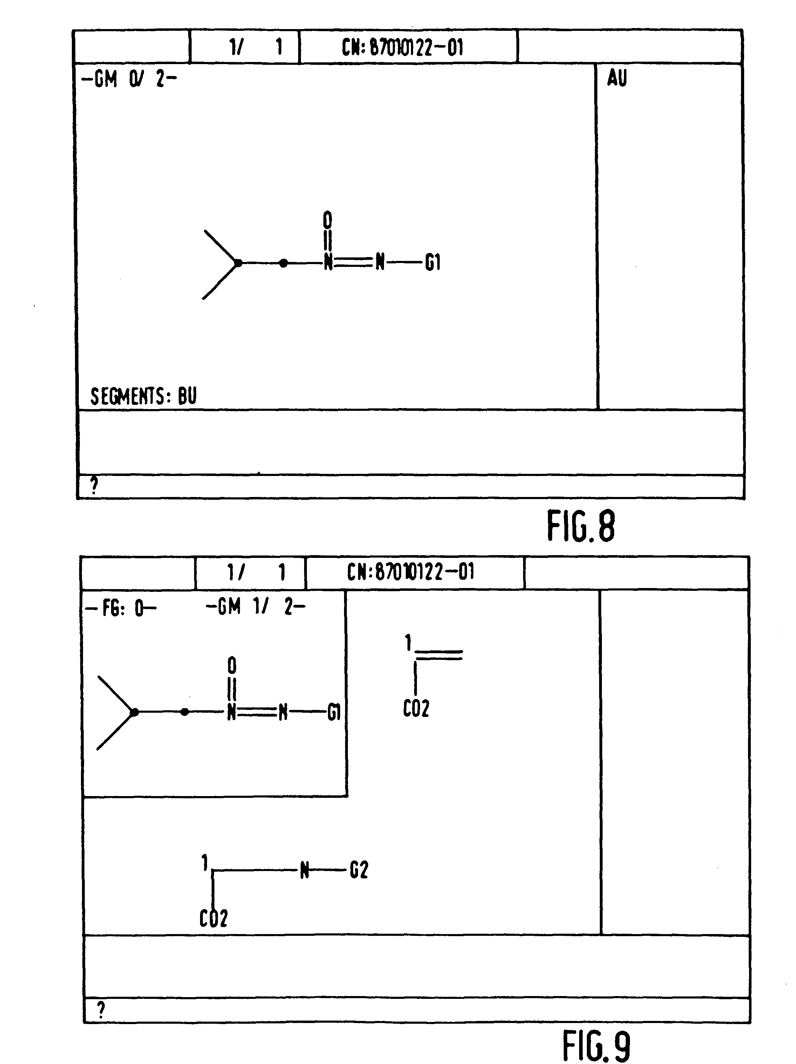

L'exemple choisi est celui d'une formule simple avantageusement saisie, visualisée ou éditée sous forme graphique comme représenté sur les figures 8 à 10.

La figure 8 montre la formule repérée par un numéro CN : 87010122-01; Elle montre l'existence d'un premier groupe G1.

La figure 9 montre le détail des valeurs possibles pour ce groupe G1, la formule générale initiale reste visible dans une fenêtre en haut à gauche de l'écran. La figure 9 montre l'existence d'un deuxième groupe G₂.

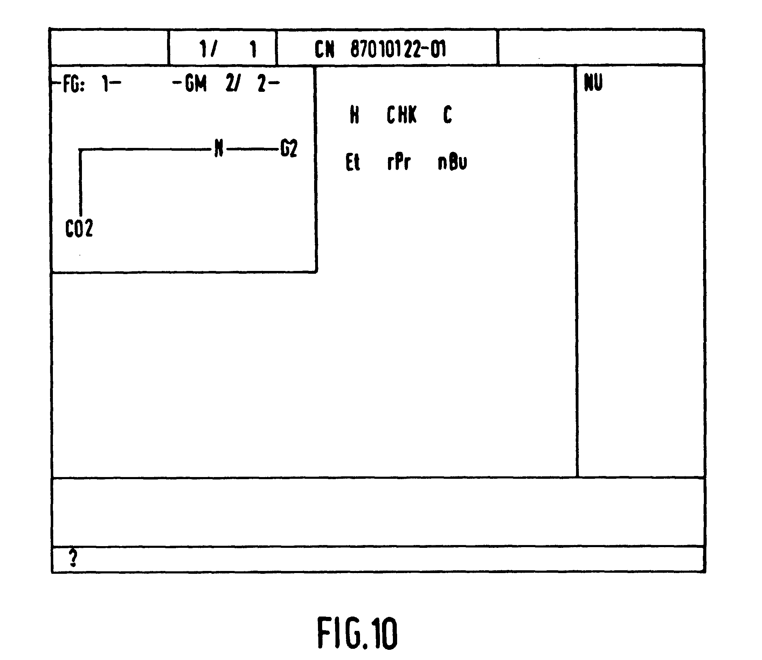

La figure 10 donne les différentes valeurs que peut prendre le groupe G₂, le groupe G₁ étant cette fois-ci visible en haut à gauche de l'écran. Ce mode de visualisation "à tiroirs" ou à fenêtre facilite notamment la recherche par l'opérateur.

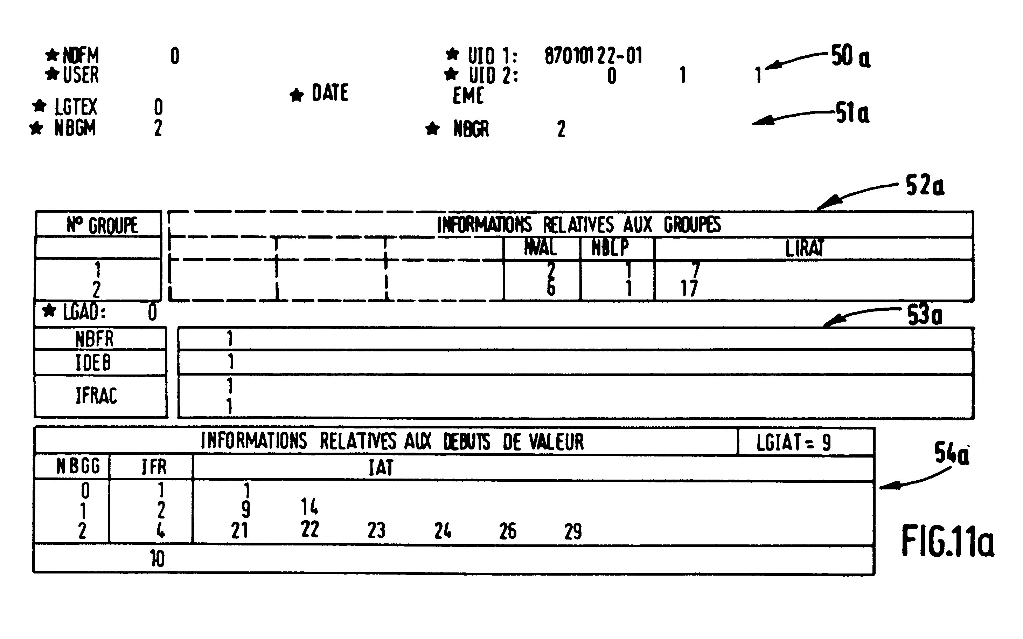

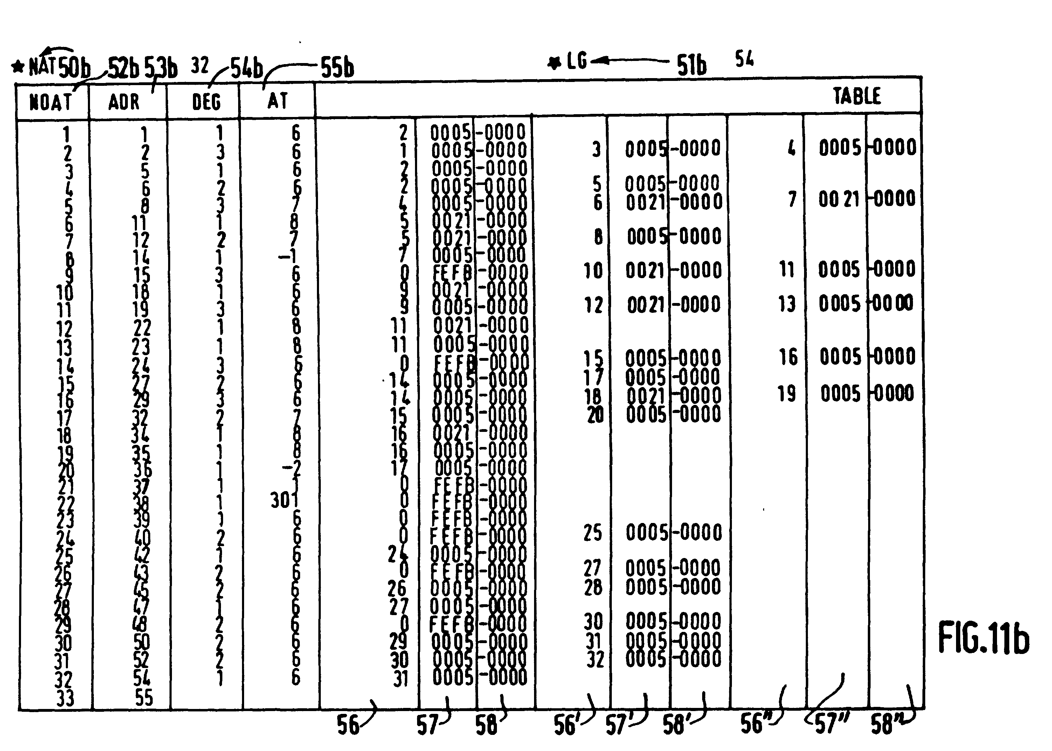

A titre d'illustration, les figures 11a et 11b donnent maintenant un exemple de table de connectivité telle que décrite dans La table VIII, ci-avant.

Il donne une bonne représentation de ce qu'on a désigné précédemment sous la dénomination "table de connectivité à tiroirs", telle qu'utilisée dans le mode de réalisation de l'invention plus particulièrement décrit ici.

Sur la figure 11a, la ligne supérieure 50a comprend essentiellement un numéro interne attribué par le logiciel (NOFM), un numéro externe attribué par l'utilisateur (UID1) et un numéro interne attribué par le logiciel à partir d'informations fournies par l'utilisateur ou l'opérateur (UID2).

La ligne 51a donne le plus grand numéro du groupe défini (NBGM) et le nombre de groupes définis (NBGR).

Le tableau 52a fait état des informations relatives aux groupes. On y retrouve notamment les tables LIRAT et NVAL mentionnées ci-avant (voir table VIII), ainsi que, par exemple et notamment une table concernant le nombre de liaisons de rattachement des groupes (NBLP)
NBLP(I) = J I = Numéro du groupe
J = Nombre de liaisons de rattachement du groupe
= - 1 pour un groupe dont les liaisons de rattachement ne sont pas définies,
= - 2 pour un groupe non existant.

Le tableau 53a donne successivement le nombre de fragment (NBFR), la table des débuts de fragments (IDEB) et la table des coefficients de fragments (IFRAC) définie par exemple de la façon suivante :
IDEB (I) = J
IFRAC (1,I) = K1
IFRAG (2,I) = K2
où I = numéro du fragment,
J = numéro d'atomes du début de fragment,
K1 = coefficient du fragment (numérateur)
K2 = coefficient du fragment (dénominateur)

IDEB (I+1)-1 étant le numéro du dernier atome du fragment I.

Le tableau 54a donne les informations relatives aux débuts de valeur. Pour chaque groupe (NBGG : numéro du groupe), sont indiqués notamment, la longueur d'IAT en mots de longueur 2 (LGIAT), la table d'adresse par groupe d'IAT (IFR), et la table de valeurs des groupes (IAT). On se réfèrera à la définition de ces tables plus haut (voir Table VIII).

La figure 11b illustre la suite des tableaux apparaissant dans le mode de réalisation de table de connectivité de l'invention plus particulièrement décrit ici.

La ligne supérieure de la table représentée sur la figure 11b, contient une indication 50b du nombre d'atomes (NAT) de la formule, et la valeur 51b de la longueur de la table (LG).

La première colonne 52b donne le N° (NOAT) attribué (arbitrairement) à chaque noeud de la formule.

La deuxième colonne 53b donne une table d'adresse (ADR) vers TABGR, TABLI et TABST (voir table VII ci-avant).

La troisième colonne 54b est le tableau donnant, pour chacun des N noeuds (ici 32) du graphe de la formule, le nombre de voisins de ce noeud.

La colonne suivante 55b est la nature de l'atome, telle que définie par la table I fournie en annexe, "-1", "-2" indiquant notamment des groupes "fils".

Les colonnes 56 (56', 56"), 57 (57', 57") et 58 (58', 58") correspondent chacune respectivement aux tableaux TABGR, TABLI et TABST définis ci-avant, dans la table VIII, pour chaque liaison du noeud concerné avec le(s) noeud(s) au(x)quel(s) il se rattache.

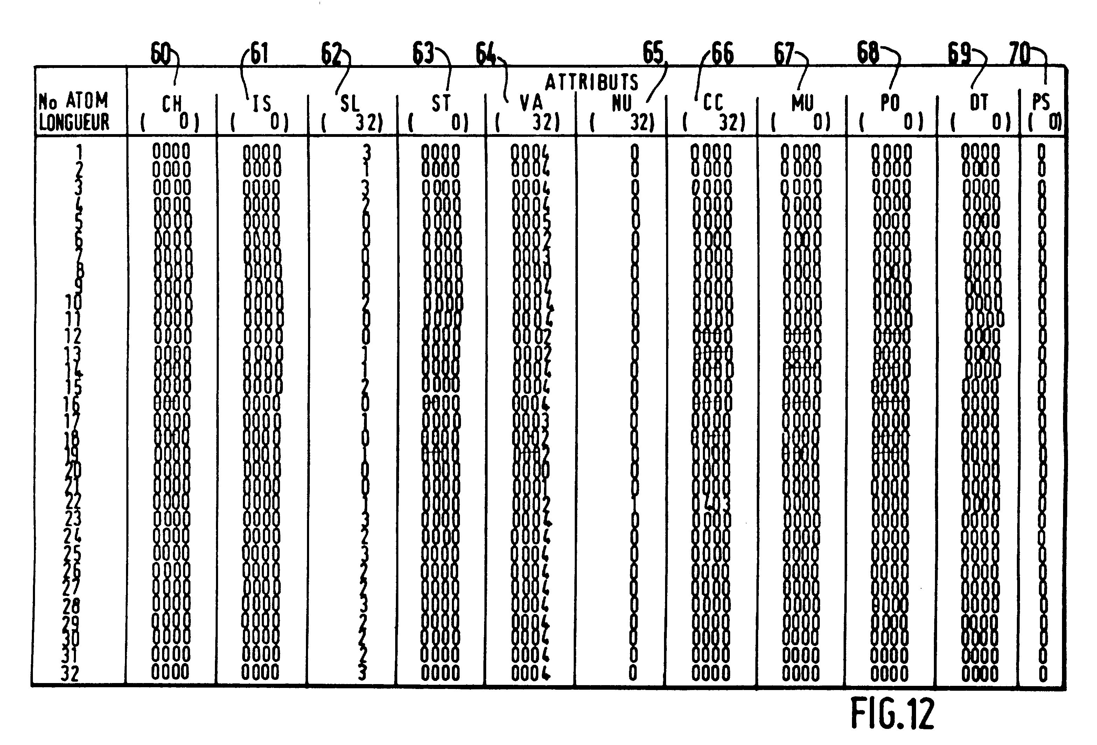

Enfin, la figure 12 représente le tableau des attributs de chacun des noeuds de la formule n° CN : 87010122-01 prise comme exemple, tel qu'il est avantageusement utilisé dans un mode de réalisation de l'invention.

On se réfèrera à la table II de définitions des attributs pour la signification des colonnes 60 à 70.

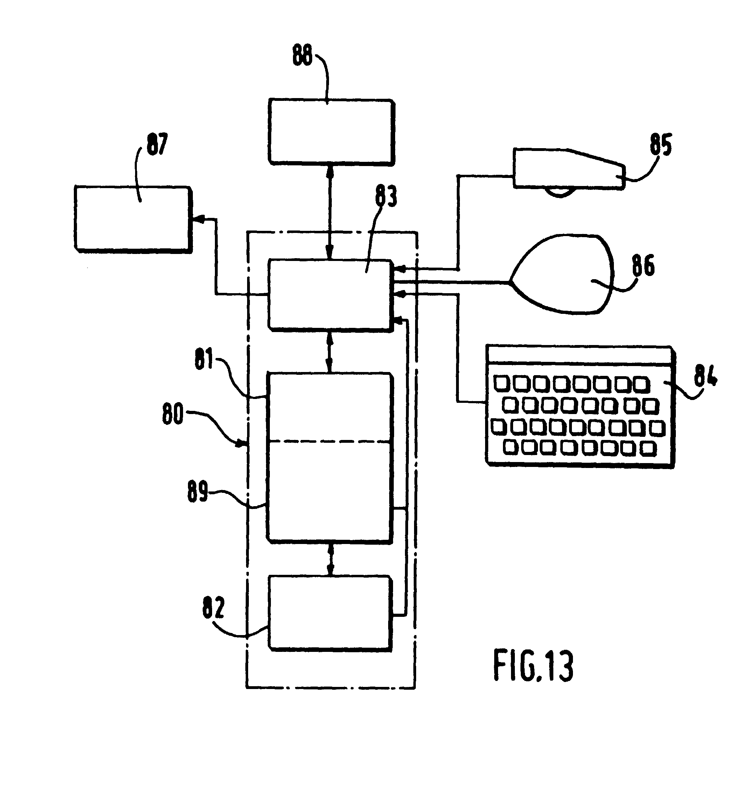

Sur la figure 13, on a représenté un système informatique selon un mode de réalisation de l'invention, mettant en oeuvre le procédé selon l'invention.

Le système comprend un ordinateur 80 du type défini plus haut, qui comporte lui même essentiellement trois éléments, une unité centrale 81, des circuits mémoires (RAM, ROM) 82 et un circuit d'interface Entrées-Sorties 83.

L'ordinateur 80 est connecté, via le circuit 83, soit à des périphériques constitués par des claviers 84, des "souris" 85 et des écrans de visualisation 86 et/ou des imprimantes, soit sur un ou des micro-ordinateurs 87.

Deux types de saisie et de recherche dans la base de donnée peuvent donc être envisagés, soi en direct, soi via un micro-ordinateur.

Une mémoire de masse 88 de grande capacité, de l'ordre du giga-octets par exemple, permettant le stockage de la base de données 14, est par ailleurs prévue, connectée à l'ordinateur 80.

L'ordinateur 80 comprend de plus des moyens 89 de génération de fichiers et de calcul, appartenant ou non à l'unité centrale 81, et comprenant un ou plusieurs microprocesseurs de type connu, par exemple des 80386 de la Société INTEL, programmés pour générer lesdits fichiers.

Ces moyens 89 sont programmés comme décrits précédemment pour générer un ficher de base (14) de formules chimiques à partir des formules de Markush introduites dans le système via les périphériques 84, 85 ou 87, et pour stocker ledit fichier de base dans la mémoire 88, sous forme de table de connectivité avec un numéro de référence et un numéro de "présélection" défini d'après la table VII, préalablement stockée en mémoire 82 ou 88.

Les moyens 89 sont également programmés pour générer pour chaque formule stockée, la liste de tous les noeuds, et pour calculer pour chaque noeud de cette liste, tous les fragments de ladite formule, issus de ce noeud.

Les moyens 89 sont enfin également programmés pour générer un fichier de fragments (18) comprenant pour chaque fragment la liste des formules incluant ledit fragment.

Cette liste est stockée dans les moyens de mémoire 82 ou 88 de type connu.

L'unité centrale 81, également programmée en conséquence, comprend par ailleurs les différents moyens d'extractions et de comparaison tels que définis précédemment et permettant d'effectuer les recherches par fragment, par numéro de sélection, noeud à noeud, par recoupement d'attributs etc... pour obtenir sur l'écran 86 ou le micro-ordinateur 87, les résultats de la recherche effectuée sur une formule de Markush donnée introduite dans le système via les périphériques 84, 85 et/ou 87.

Les moyens 89 pour générer le fichier de base 14, comprennent un ou des circuits électroniques programmés pour constituer un numéro de présélection sous forme d'une chaîne de n bits, dont chaque bit correspond à un caractère type de la table de définition (table VII).

On va maintenant décrire sommairement ci-après la mise en oeuvre par un opérateur, du procédé et du système de recherche d'une formule de Markush, selon le mode de réalisation de l'invention plus particulièrement décrit ici.

L'opérateur se connecte via les périphériques 84, 85 et 86, ou 87, à l'ordinateur central 80 raccordé à la mémoire 88 contenant la base de donnée 14.

Il introduit sa question dans le système sous forme alphanumérique ou graphique en définissant la formule par l'intermédiaire d'un clavier ou d'une "souris" raccordée à l'ordinateur.

Pour ce faire on a vu qu'il utilisait d'une part un programme d'application de saisie graphique et/ou alphanumérique connu, et un programme 5 de mise en forme des formules de Markush sous une forme déterminée selon l'invention (table de connectivité..).

L'opérateur complète éventuellement sa question en précisant les attributs, voire d'autre paramètres comme par exemple la période sur laquelle la recherche doit être effectuée. Dans ce dernier cas, l'opérateur limite alors sa question aux formules apparaissant dans la littérature sur une période donnée ( à partir de 1980 par exemple), etc...

Le système calcule ensuite automatiquement les fragments de la formule recherchée, puis effectue la première recherche sur fragments en faisant apparaître sur l'écran le nombre de candidats trouvé ayant un fragment commun avec la formule recherchée, nombre qui peut d'ailleurs être nul.

Si l'opérateur le souhaite, il appuie sur une touche pour effectuer la deuxième recherche sur fragment et ainsi de suite.

Une fois que l'opérateur estime que la liste obtenue est la liste "issue de fragments" de longueur optimisée, ou encore que ladite comparaison ne permet plus de réduire le nombre de formules de la liste "issue de fragments" de façon significative, ou par rapport à un critère prédéterminé, l'opérateur, ou le système informatique, commande la comparaison des numéros de présélection, et la suite de la recherche pour obtenir in fine et éventuellement, une liste de formules candidats, ou le candidat recherché, après avoir choisi ou non d'effectuer une recherche noeud par noeud.

Il peut ensuite faire apparaître à l'écran la ou les formules candidats.

Comme il va de soi, et comme il résulte d'ailleurs de ce qui précède, la présente invention ne se limite nullement aux modes de réalisation plus particulièrement décrits. Elle comprend notamment les variantes de réalisation faisant intervenir des programmes équivalents, dont les modifications par rapport aux exemples donnés, sont à la portée de l'homme du métier.

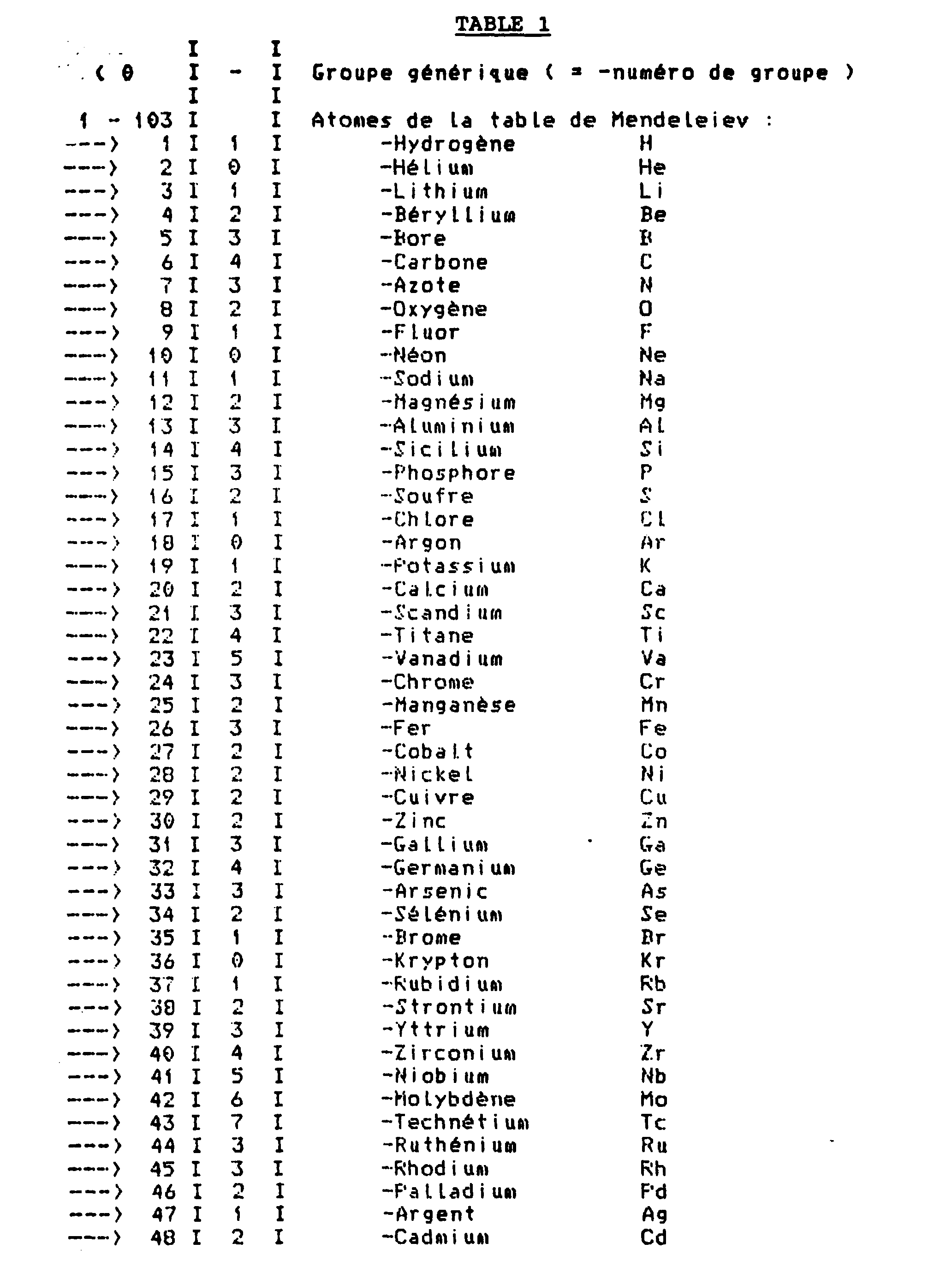

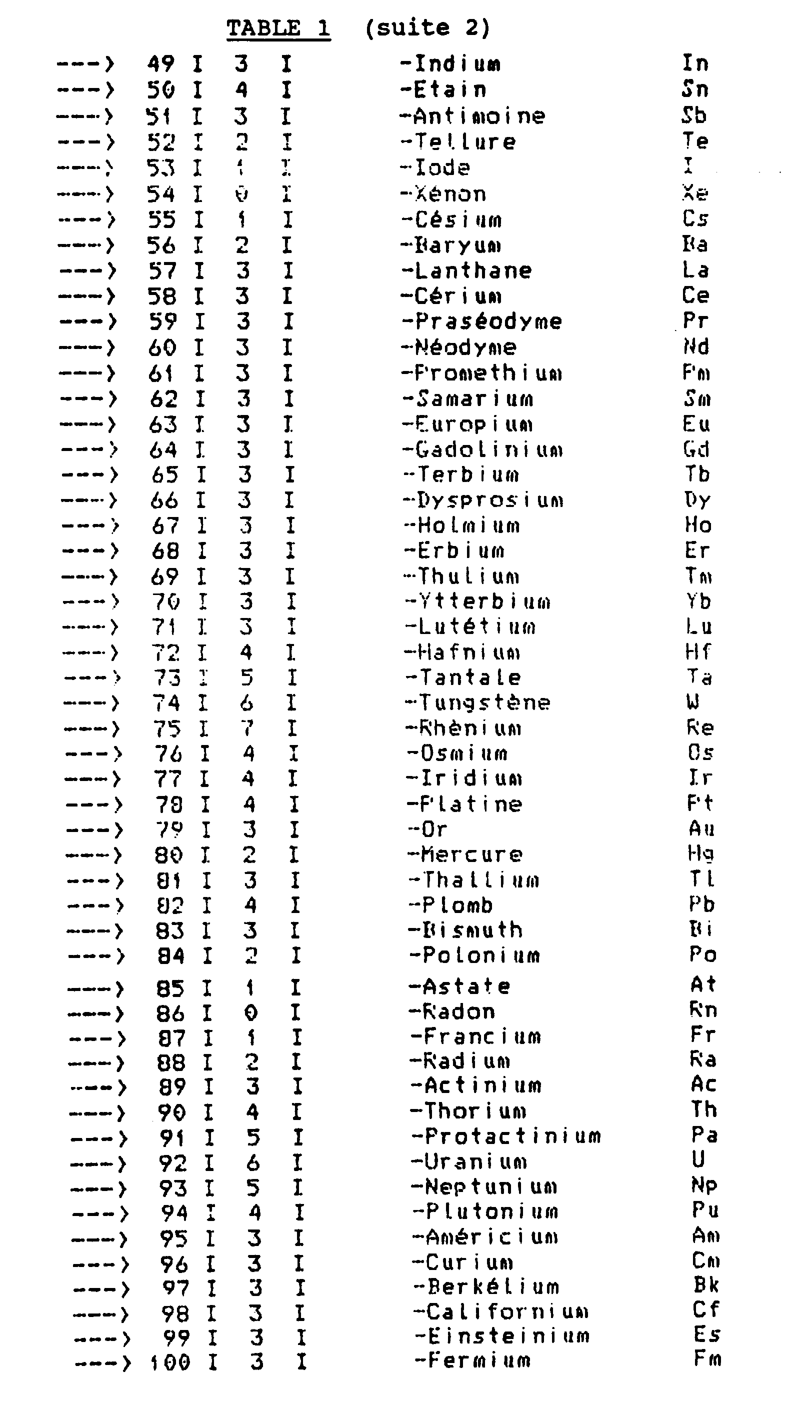

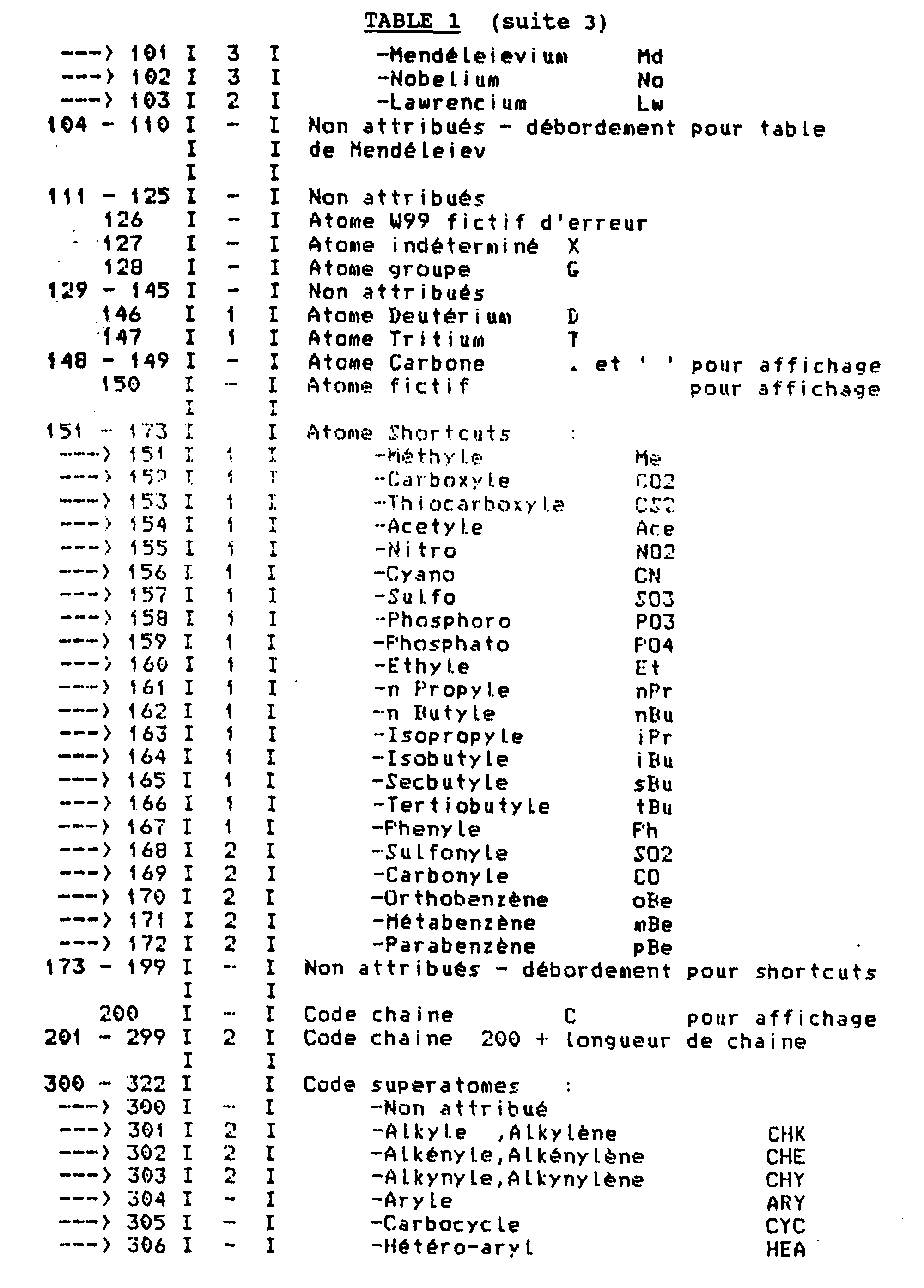

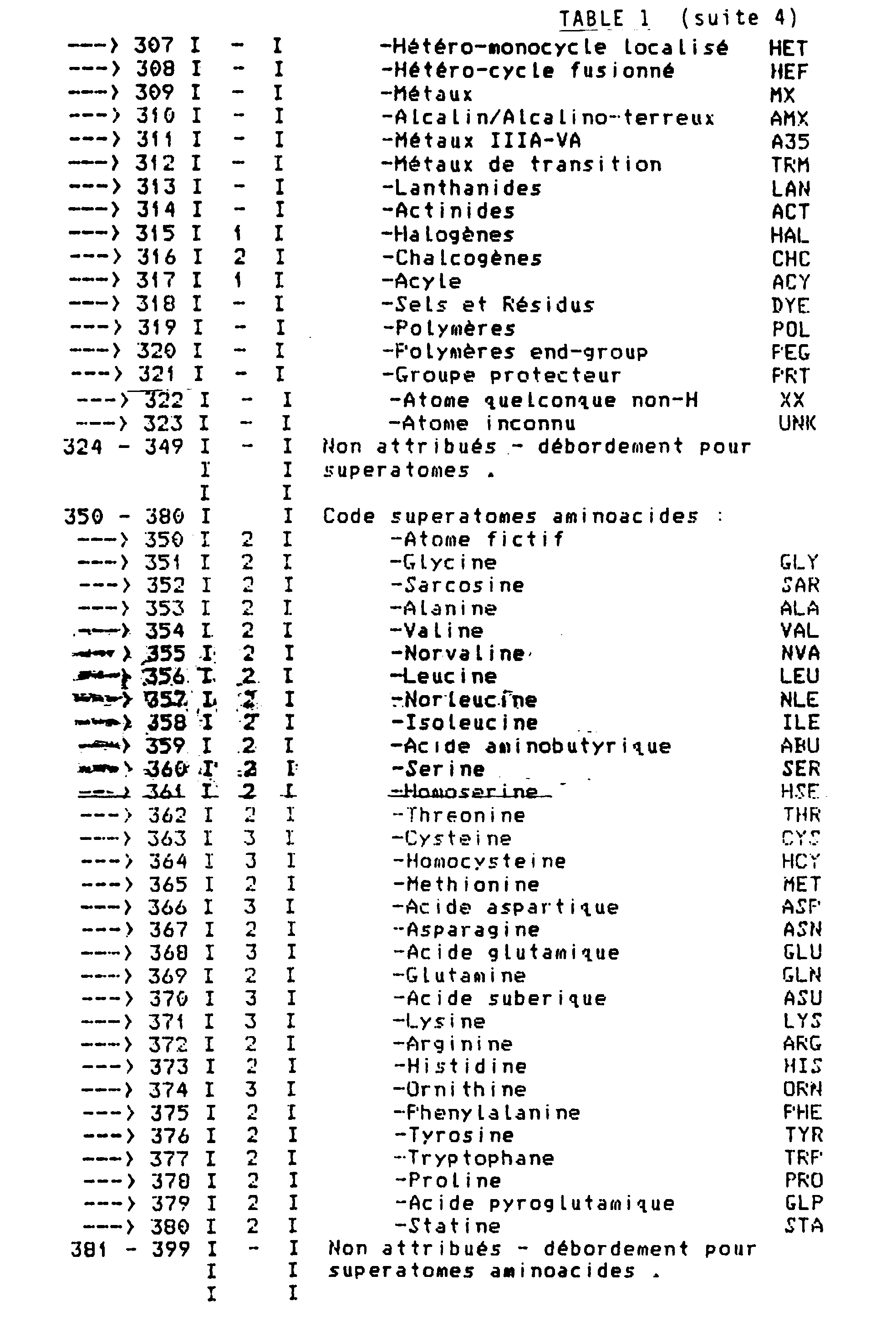

## Revendications

1. Procédé de stockage et de recherche de formules de Markush (1), caractérisé en ce que
a) un fichier de base (14) de formules chimiques est formé à partir desdites formules de Markush, dans lequel chaque formule de Markush est stockée sous forme de tables de connectivité (TC₁, TC₂; ... TCₙ) comprenant un numéro de référence (NOFM₁, NOFM₂, ..., NOFMₙ) de ladite formule stockée et un numéro de "présélection" (Nps₁, Nps₂, ..., Npsₙ) décrivant synthétiquement des caractères types prédéterminés de ladite formule, définis selon une table de définition préalablement constituée desdits caractères types (table VII),
b) pour chaque formule du fichier de base (14) et pour la formule recherchée (3) stockée sous forme identique aux formules du fichier, la liste de tous les noeuds ou atomes foyers comprenant au moins deux voisins immédiats est déterminée et, un "fragment" étant défini comme une partie de structure de formule constituée par un foyer, ses atomes voisins immédiats (environnement 1) et s'ils existent le ou les atomes voisins immédiats du foyer (environnement 2) de chacun des atomes voisins immédiats du foyer, pour chaque noeud de cette liste, toutes les parties possibles, ou fragments, de ladite formule issues de ce noeud sont calculées,
c) un fichier de fragments (18) est formé comprenant, pour chaque fragment calculé des formules du fichier de base, la liste des numéros de références desdites formules comprenant ce fragment,
d) pour un premier fragment de la formule de Markush recherchée, une première liste de numéros de référence des formules possibles comprenant ledit fragment de ladite formule de Markush recherchée est extraite dudit fichier de fragments (18),
e) puis s'il y a lieu et pour un second fragment de la formule recherchée, une deuxième liste de numéros de référence des formules possibles comprenant ledit deuxième fragment est extraite dudit fichier de fragments (18) ; cette deuxième liste est comparée à la première liste obtenue pour le premier fragment, pour former une troisième liste "issue des fragments", constituée par les numéros communs des deux premières listes,
et l'opération est réitérée tant qu'il est estimé nécessaire en comparant chaque nouvelle liste de numéros de formules possibles correspondant à un nouveau fragment avec la liste constituée par les numéros communs de toutes les listes précédentes, pour former une liste "issue de fragments" de longueur optimisée,
f) le numéro de "présélection" de la formule de Markush recherchée est comparé aux numéros de "présélection" des formules de la liste "issue de fragments" de longueur optimisée, et une liste finale en est extraite, formée par les numéros de référence des formules ayant un numéro de "présélection" identique à celui de la formule de Markush recherchée.

2. Procédé de stockage et de recherche de formules de Markush (1) selon la revendication 1, caractérisé en ce que partant d'une base de données (6) comprenant le fichier de base (14) de formules chimiques formé à partir de formules de Markush (1), et le fichier de fragments (18) comprenant les listes des numéros de références des formules associées à chaque fragment des formules du fichier de base (14), la formule de Markush recherchée (3) est stockée sous forme similaire aux formules de la base de données (6), c'est-à-dire sous forme de table de connectivité (TCᵢ) avec un numéro de présélection (Npsi) de ladite formule recherchée.

3. Procédé de stockage et de recherche de formules de Markush (1) par un opérateur selon la revendication 1, caractérisé en ce que chaque formule de Markush du fichier de base (14) est stockée dans un système informatique par l'opérateur,
en ce que la liste de tous les noeuds ou atomes foyers comprenant au moins deux voisins immédiats est déterminée automatiquement pour chaque formule du fichier de base (14) et pour la formule recherchée (3) stockée sous forme identique aux formules du fichier par l'opérateur,
en ce que toutes les parties possibles, ou fragments, de ladite formule issues de ce noeud sont calculées automatiquement pour chaque noeud de cette liste,
en ce que le fichier de fragments (18) est formé automatiquement,
en ce que la deuxième liste de numéros de référence des formules possibles comprenant ledit deuxième fragment est extraite suite à une commande de l'opérateur et comparée automatiquement avec la première liste obtenue pour le premier fragment,
en ce que l'opération est réitérée par commande manuelle de l'opérateur
en comparant automatiquement chaque nouvelle liste de numéros de formules possibles correspondant à un nouveau fragment avec la liste constituée par les numéros communs de toutes les listes précédentes, pour former automatiquement une liste "issue de fragments" de longueur optimisée,
et en ce que suite à une commande manuelle de l'opérateur, le numéro de "présélection" de la formule de Markush recherchée est comparé automatiquement aux numéros de "présélection" des formules de la liste "issue de fragments" de longueur optimisée, la liste finale en étant extraite automatiquement.

4. Procédé de stockage et de recherche de formules de Markush (1) par un utilisateur, dans un système informatique selon les revendications 2 et 3, caractérisé en ce que chaque formule de Markush est stockée dans le système informatique sous la forme des tables de connectivité (TCᵢ),
en ce que l'utilisateur stocke dans le système informatique la formule de Markush recherchée (3) sous forme similaire aux formules de la base de données (6),
en ce que le système informatique détermine automatiquement pour la formule recherchée (3), la liste des noeuds et calcule tous les fragments,
en ce que le système informatique extrait automatiquement dudit fichier de fragments (18) la première liste de numéros de référence,
en ce que le système informatique extrait automatiquement dudit fichier de fragments (18) la deuxième liste de numéros de référence que le système informatique compare avec la première liste obtenue pour le premier fragment, pour former la troisième liste,
en ce que le système informatique réitère l'opération initiée par commande manuelle de l'utilisateur, tant que l'utilisateur l'estime nécessaire, en comparant automatiquement chaque nouvelle liste avec la liste constituée par les numéros communs de toutes les listes précédentes,
et en ce que, suite à une commande manuelle de l'opérateur, le système informatique compare automatiquement le numéro de "présélection" aux numéros de "présélection" des formules de la liste "issue de fragments" et en extrait la liste finale.

5. Procédé de stockage et de recherche de formules de Markush (1) par un utilisateur dans un système informatique selon les revendications 2 et 3, caractérisé en ce que, partant de la base de données (6) du système informatique, l'utilisateur introduit au moins partiellement la formule de Markush recherchée (3) sous une forme graphique déterminée dans le système informatique,
en ce que le système informatique lit ladite représentation graphique et la transforme pour la stocker,
en ce que l'opération de formation de la troisième liste est réitérée par le système informatique qui compare automatiquement chaque nouvelle liste avec la liste constituée par les numéros communs de toutes les listes précédentes, jusqu'à ce que ladite comparaison ne permette plus de réduire le nombre de formules de la liste "issue de fragments" de façon significative, pour former une liste issue de fragments de longueur optimisée,
en ce que le système informatique, en automatique, ou l'utilisateur, en manuel, commande ensuite l'étape où ledit système informatique compare le numéro de "présélection" aux numéros de "présélection" des formules de la liste "issue de fragments", et en extrait la liste finale,
et en ce que le système informatique extrait, une à une, à la demande de l'utilisateur, et s'il y a lieu, les formules détenues dans ladite liste finale, en les transformant pour les représenter sous forme d'une image graphique adaptée propre à permettre une comparaison optimisée avec la forme graphique déterminée de la formule recherchée.

6. Procédé selon l'une quelconque des revendications précédentes, caractérisé en ce que on effectue de plus une comparaison noeud à noeud des formules de ladite liste finale, avec la formule de Markush recherchée (3), et on extrait les références de la ou des formules de Markush éventuellement obtenues par cette comparaison.

7. Procédé selon l'une quelconque des revendications précédentes, caractérisé en ce que l'on affecte à certains des atomes ou groupes d'atomes des formules de Markush du fichier de base (14) interrogé et s'il y a lieu à la formule de Markush recherchée (3), un ensemble d'attributs, et on ne recherche dans ledit fichier de base de formules de Markush que les formules comportant une structure munie du ou des attributs spécifiquement affectés à la formule recherchée.

8. Procédé selon l'une quelconque des revendications précédentes, caractérisé en ce que l'on stocke les formules de Markush constituées par des graphes, en utilisant quand nécessaire aux noeuds desdits graphes, de faux atomes représentant des termes génériques et/ou des ensembles d'atomes invariants pour simplifier les représentations desdites formules.

9. Procédé selon l'une quelconque des revendications précédentes, caractérisé en ce que l'on stocke les formules de Markush dans le fichier de base (14) et on interroge ledit fichier de base pour rechercher une formule de Markush spécifique, sous forme essentiellement graphique, à l'aide d'une interface du type "souris".

10. Procédé selon l'une quelconque des revendications précédentes, caractérisé en ce que l'on constitue le numéro de "présélection" (Npsi), décrivant synthétiquement des caractères types prédéterminés d'une formule de Markush, sous forme d'une chaîne de n bits, dont chaque bit correspond à un caractère type déterminé de la table de définition préalablement constituée desdits n caractères types (Table VII).

11. Procédé selon l'une quelconque des revendications précédentes, caractérisé en ce que les tables de connectivité représentant les formules de Markush sont constituées par des tables de connectivité à "tiroir" comprenant
- quatre tables donnant les relations entre les différents groupes d'atomes, ou graphes "fils" de la structure, constituées par une première table (LIRAT) identifiant les atomes auxquels se rattachent lesdits groupes,
- une deuxième table (NVAL) donnant les nombres de valeurs pour chacun desdits groupes,
- une troisième table (IAT) donnant les débuts de valeurs desdits groupes et,
- une quatrième table (IFR) donnant les adresses par groupe vers la table des débuts de valeurs (IAT).

12. Système informatique pour la mise en oeuvre du procédé de stockage et de recherche de formules de Markush selon l'une quelconque des revendications précédentes, comprenant une unité centrale (81), des mémoires de travail et de stockage (82, 88), des mémoires d'entrées (84, 85, 87) de formules de Markush sous forme graphique et/ou alpha-numérique et des moyens de restitution d'informations (86, 87) caractérisé en ce qu'il comprend
- des moyens (81, 89) pour générer un fichier de base (14) de formules chimiques, à partir desdites formules de Markush, sous forme de tables de connectivité (TCᵢ), chaque formule comprenant un numéro de référence (NOFMᵢ) et un numéro de "présélection" (Npsi) décrivant des caractères types de ladite formule définis selon une table de définition (table VII) préalablement stockée dans une mémoire (88) du système,
- des moyens pour générer (81, 89), pour chaque formule stockée ou recherchée, la liste de tous les noeuds, ou atomes foyers, comprenant au moins deux voisins immédiats,
- des moyens de calcul (89), pour chaque noeud de cette liste, de toutes les parties possibles, dites fragments, de ladite formule, issues de ce noeud, un fragment étant défini comme une partie de structure de formule constituée par un foyer, ses atomes voisins immédiats (environnement 1) et s'ils existent le ou les atomes voisins immédiats (environnement 2) de chacun des atomes voisins immédiats du foyer,
- des moyens pour générer (81, 89) un fichier de fragments comprenant, pour chaque fragment calculé des formules du fichier de base, la liste des numéros de références des formules comprenant ce fragment,
- des moyens d'extraction (81), à partir dudit fichier de fragments, de listes de numéros de référence de formules possibles comprenant au moins un fragment de ladite formule de Markush recherchée, lesdits moyens étant prévus pour générer une liste "issue de fragments" de longueur optimisée constituée par les numéros de Markush comportant un nombre optimisé, déterminé par l'utilisateur, de fragments communs avec la formule de Markush recherchée,
- et des moyens de comparaison (81) du numéro de "présélection" de la formule de Markush recherchée avec les numéros de "présélection" des formules de la liste "issue de fragments" de longueur optimisée et à partir de cette comparaison, de constitution d'une liste finale formée par les numéros de références des formules ayant un numéro de "présélection" identique à celui de la formule de Markush recherchée.

13. Système selon la revendication 12, caractérisé en ce qu'il comporte de plus des moyens de comparaison noeud à noeud des formules de ladite liste finale avec la formule de Markush recherchée, et d'extraction des références de la ou des formules de Markush éventuellement obtenues par cette comparaison.

14. Système selon l'une quelconque des revendications 12 et 13, caractérisé en ce que les moyens pour générer un fichier de formules chimiques à partir desdites formules de Markush sous forme de table de connectivité, comprennent des moyens de constitution d'un numéro de présélection sous forme d'une chaîne de n bits, dont chaque bit correspond à un caractère type déterminé de la table de définition préalablement constituée desdits n caractères types (table VII).

## Patentansprüche

1. Verfahren zur Speicherung und Suche von Markush-Formeln (1),
dadurch gekennzeichnet, daß
(a) eine Basisdatei (14) chemischer Formeln ausgehend von den Markush-Formeln gebildet wi.rd, in welcher Basisdatei jede Markush-Formel in Form von Konnektivitätstabellen (TC₁, TC₂, ... TCₙ) gespeichert ist, die eine Bezugsnummer (NOFM₁, NOFM₂, ..., NOFMₙ) der gespeicherten Formel und eine "Vorauswahl"-Nummer (Nps₁, Nps₂, ..., Npsₙ) enthalten, die vorbestimmte Typzeichen der Formel synthetisch beschreibt, die gemäß einer zuvor aus den Typzeichen (Tabelle VII) gebildeten Definitionstabelle definiert sind,
b) für jede Formel der Basisdatei (14) und für die gesuchte Formel (3), die identisch mit den Formeln der Datei gespeichert ist, die Liste aller Knoten oder Herd-Atome mit mindestens zwei unmittelbaren Nachbarn bestimmt wird und, wobei ein "Fragment" als ein Teil der Struktur der Formel definiert ist, die durch einen Herd, seine unmittelbar benachbarten Atome (Umgebung 1) und, falls vorhanden, das unmittelbar benachbarte Atom oder die unmittelbar benachbarten Atome des Herds (Umgebung 2) jedes der dem Herd unmittelbar benachbarten Atome gebildet ist, für jeden Knoten dieser Liste alle möglichen Teile oder Fragmente der von diesem Knoten abgeleiteten Formel berechnet werden,
c) eine Fragmentedatei (18) gebildet wird, die für jedes berechnete Fragment der Formeln der Basisdatei die Liste der Bezugsnummern der dieses Fragment enthaltenden Formeln enthält,
d) für ein erstes Fragment der gesuchten Markush-Formel eine erste Liste von Bezugsnummern der möglichen Formeln, die das Fragment der gesuchten Markush-Formel enthalten, aus der Fragmentedatei (18) extrahiert wird,
e) anschließend notwendigenfalls und für ein zweites Fragment der gesuchten Formel eine zweite Liste von Bezugsnummern der möglichen Formeln, die das zweite Fragment enthalten, aus der Fragmentedatei (18) extrahiert wird; diese zweite Liste mit der ersten, für das erste Fragment erhaltenen Liste verglichen wird, um eine dritte, "von den Fragmenten abgeleitete" Liste zu bilden, die aus den gemeinsamen Nummern der ersten beiden Listen gebildet ist,
und die Operation solange wiederholt wird, wie sie für notwendig erachtet wird, indem jede neue Nummernliste möglicher Formeln, die einem neuen Fragment entsprechen, mit der Liste verglichen wird, die aus den gemeinsamen Nummern aller vorhergehenden Listen gebildet ist, um eine "von Fragmenten" einer optimierten Länge "abgeleitete" Liste zu bilden,
f) die "Vorauswahl"-Nummer der gesuchten Markush-Formel mit den "Vorauswahl"-Nummern der Formeln der "von Fragmenten" optimierter Länge "abgeleiteten" Liste verglichen wird und daraus eine abschließende Liste extrahiert wird, die aus den Bezugsnummern der Formeln gebildet ist, die eine "Vorauswahl"-Nummer aufweisen, die mit derjenigen der gesuchten Markush-Formel identisch ist.

2. Verfahren zur Speicherung und Suche von Markush-Formeln (1) nach Anspruch 1, dadurch gekennzeichnet, daß ausgehend von einer Datenbank (6), die die Basisdatei (14) chemischer Formeln, die ausgehend von Markush-Formeln (1) gebildet ist, und die Fragmentedatei (18) enthält, die die Listen der Bezugsnummern der mit jedem Fragment der Formeln der Basisdatei (14) assoziierten Formeln enthält, die gesuchte Markush-Formel (3) in ähnlicher Form gespeichert wird wie die Formeln der Datenbank (6), d. h. in Form einer Konnektivitätstabelle (TCᵢ) mit einer Vorauswahl-Nummer (Npsi) der gesuchten Formel.

3. Verfahren nach Anspruch 1 zur Speicherung und Suche von Markush-Formeln (1) durch eine Bedienungsperson,
dadurch gekennzeichnet,
daß jede Markush-Formel der Basisdatei (14) in einem Datenverarbeitungssystem durch die Bedienungsperson gespeichert wird,
daß die Liste aller Knoten oder Herdatome mit mindestens zwei unmittelbaren Nachbarn für jede Formel der Basisdatei (14) und für die gesuchte Formel (3) automatisch bestimmt wird, die identisch mit den Formeln der durch die Bedienungsperson gespeicherten Datei gespeichert ist,
daß alle möglichen Teile oder Fragmente der Formel, die von diesem Knoten abgeleitet sind, automatisch für jeden Knoten dieser Liste berechnet werden,
daß die Fragmentedatei (18) automatisch gebildet wird, daß die zweite Liste von Bezugsnummern der möglichen, das zweite Fragment enthaltenden Formeln infolge eines Befehls der Bedienungsperson extrahiert wird und mit der ersten, für das erste Fragment erhaltenen Liste verglichen wird,
daß die Operation durch manuellen Befehl der Bedienungsperson wiederholt wird, indem jede neue Nummernliste möglicher, einem neuen Fragment entsprechender Formeln mit der Liste verglichen wird, die aus den Nummern gebildet ist, die allen vorhergehenden Listen gemeinsamen sind, um eine "von Fragmenten" optimierter Länge "abgeleitete" Liste automatisch zu bilden,
und daß infolge eines manuellen Befehls der Bedienungsperson die "Vorauswahl"-Nummer der gesuchten Markush-Formel mit den "Vorauswahl"-Nummern der Formeln der Liste automatisch verglichen wird, die "von Fragmenten" optimierter Länge "abgeleitet" ist, wobei die abschließende Liste daraus automatisch extrahiert wird.

4. Verfahren zur Speicherung und Suche von Markush-Formeln (1) durch einen Benutzer in einem Datenverarbeitungssystem nach Anspruch 2 und 3,
dadurch gekennzeichnet, daß jede Markush-Formel in dem Datenverarbeitungssystem in Form von Konnektivitätstabellen (TCᵢ) gespeichert wird,
daß der Benutzer in dem Datenverarbeitungssystem die gesuchte Markush-Formel (3) ähnlich wie die Formeln der Datenbank (6) speichert,
daß das Datenverarbeitungssystem für die gesuchte Formel (3) die Liste der Knoten automatisch bestimmt und alle Fragmente berechnet,
daß das Datenverarbeitungssystem automatisch aus der Fragmentedatei (18) die erste Bezugsnummern-Liste extrahiert,
daß das Datenverarbeitungssystem automatisch aus der Fragmentedatei (18) die zweite Bezugsnummern-Liste extrahiert, die das Datenverarbeitungssystem mit der ersten, für das erste Fragment erhaltenen Liste vergleicht, um die dritte Liste zu bilden,
daß das Datenverarbeitungssystem die durch manuellen Befehl des Benutzers eingeleitete Operation solange wiederholt, wie es der Benutzer für notwendig erachtet, indem jede neue Liste mit der Liste automatisch verglichen wird, die aus den Nummern gebildet ist, die allen vorhergehenden Listen gemeinsam sind,
daß infolge eines manuellen Befehls der Bedienungsperson das Datenverarbeitungssystem automatisch die "Vorauswahl"-Nummer mit den "Vorauswahl"-Nummern der Formeln der Liste vergleicht, die "von Fragmenten abgeleitet ist", und daraus die abschließende Liste extrahiert.

5. Verfahren nach Anspruch 2 und 3 zur Speicherung und Suche von Markush-Formeln (1) durch einen Benutzer in einem Datenverarbeitungssystem,
dadurch gekennzeichnet, daß ausgehend von der Datenbank (6) des Datenverarbeitungssystems der Benutzer mindestens teilweise die gesuchte Markush-Formel (3) in graphischer Form eingibt, die in dem Datenverarbeitungssystem bestimmt ist,
daß das Datenverarbeitungssystem die graphische Darstellung liest und sie transformiert, um sie zu speichern, daß die Operation der Bildung der dritten Liste durch das Datenverarbeitungssystem wiederholt wird, das jede neue Liste mit der Liste automatisch vergleicht, die aus den Nummern gebildet ist, die allen vorhergehenden Listen gemeinsamen sind, bis der Vergleich nicht mehr gestattet, die Anzahl von Formeln der "von Fragmenten abgeleiteten" Liste signifikativ zu reduzieren, um eine Liste zu bilden, die von Fragmenten optimierter Länge abgeleitet ist,
daß das Datenverarbeitungssystem automatisch oder der Benutzer manuell daraufhin den Schritt steuert, wo das Datenverarbeitungssystem die "Vorauswahl"-Nummer mit den "Vorauswahl"-Nummern der Formeln der Liste vergleicht, die "von Fragmenten abgeleitet" ist, und daraus die abschließende Liste extrahiert,
und daß das Datenverarbeitungssystem eins zu eins auf Anforderung des Benutzers und notwendigenfalls die in der abschließenden Liste enthaltenen Formeln extrahiert, indem sie transformiert werden, um sie in Form eines adaptierten graphischen Bilds darzustellen, das geeignet ist, einen optimierten Vergleich mit bestimmten graphischen Form der gesuchten Formel zu gestatten.

6. Verfahren nach einem der vorhergehenden Ansprüche, dadurch gekennzeichnet, daß ferner ein Knoten-zu-Knoten-Vergleich der Formeln der abschließenden Liste mit der gesuchten Markush-Formel (3) ausgeführt wird und daß die Bezugspunkte bzw. Referenzen der Markush-Formel oder der Markush-Formeln extrahiert werden, die gegebenenfalls durch diesen Vergleich erhalten wurden.

7. Verfahren nach einem der vorhergehenden Ansprüche, dadurch gekennzeichnet, daß bestimmten Atomen oder Atomgruppen der Markush-Formeln der abgefragten Basisdatei (14), und notwendigenfalls der gesuchten Markush-Formel (3) eine Einheit von Attributen zugewiesen wird und in der Markush-Formeln-Basisdatei nur die Formeln gesucht werden, die eine Struktur aufweisen, die mit dem Attribut oder Attributen versehen ist, das bzw. die der gesuchten Formel spezifisch zugewiesen ist bzw. sind.

8. Verfahren nach einem der vorhergehenden Ansprüche, dadurch gekennzeichnet, daß die durch Graphen gebildeten Markush-Formeln gespeichert werden, indem, wenn für die Knoten der Graphen erforderlich, falsche Atome verwendet werden, die Gattungsbegriffe und/oder Einheiten invarianter Atome darstellen, um die Darstellungen der Formeln zu vereinfachen.

9. Verfahren nach einem der vorhergehenden Ansprüche, dadurch gekennzeichnet, daß die Markush-Formeln in der Basisdatei (14) gespeichert werden und die Basisdatei abgefragt wird, um eine spezifische Markush-Formel, im wesentlichen in graphischer Form mithilfe einer Schnittstelle vom "Maus"-Typ zu suchen.

10. Verfahren nach einem der vorhergehenden Ansprüche, dadurch gekennzeichnet, daß die "Vorauswahl"-Nummer (Npsi), die vorbestimmte Typzeichen einer Markush-Formel synthetisch beschreibt, in Form einer Kette von n Bits gebildet wird, von denen jedes Bit einem bestimmten Typzeichen der Definitionstabelle entspricht, die zuvor aus n Typzeichen gebildet wurde (Tabelle VII).

11. Verfahren nach einem der vorhergehenden Ansprüche, dadurch gekennzeichnet, daß die Konnektivitätstabellen, die die Markush-Formeln darstellen, aus "Schubladen"-Konnektivitätstabellen gebildet werden, aufweisend:
- Vier Tabellen, die die Beziehungen zwischen den verschiedenen Atomgruppen oder "Neben"-Graphen (graphes "fils") der Struktur wiedergeben, welche Tabellen aus einer ersten Tabelle (LIRAT) gebildet sind, die die Atome kennzeichnet, mit denen die Gruppen in Verbindung stehen, - eine zweite Tabelle (NVAL), die die Werte-Ziffern (nombres de valeurs) für jede der Gruppen angibt,
- eine dritte Tabelle (IAT), die die Anfänge der Werte der Gruppen angibt und
- eine vierte Tabelle (IFR), die die Adressen pro Gruppe an die Tabelle der Werteanfänge (IAT) liefert.

12. Datenverarbeitungssystem für den Einsatz des Verfahrens zur Speicherung und Suche von Markush-Formeln nach einem der vorhergehenden Ansprüche, aufweisend eine Zentraleinheit (81), Arbeits- und Pufferspeicher (82, 88), Speicher (84, 85, 87) für die Eingabe von Markush-Formeln in graphischer und/oder alphanumerischer Form und Mittel zur Datenwiedergabe (86, 87),
dadurch gekennzeichnet, daß es umfaßt:
- Mittel (81, 89), um eine Basisdatei (14) chemischer Formeln ausgehend von den Markush-Formeln in Form von Konnektivitätstabellen (TC₁) zu erzeugen, wobei jede Formel eine Bezugsnummer (NOFM) und eine "Vorauswahl"-Nummer (Npsi) enthält, die Typzeichen der Formel beschreibt, die gemäß einer Definitionstabelle (Tabelle VII) definiert sind, die zuvor in einem Speicher (88) des Systems gespeichert wurde,
- Mittel (81, 89), um für jede gespeicherte oder gesuchte Formel die Liste alle Knoten oder Herd-Atome mit mindestens zwei unmittelbaren Nachbarn zu erzeugen,
- Mittel (89) zur Berechnung aller möglichen Teile der Formel, Fragmente genannt, die von diesem Knoten abgeleitet sind, für jeden Knoten dieser Liste, wobei ein Fragment als ein Teil der Struktur der Formel definiert ist, die durch einen Herd, seine unmittelbar benachbarten Atome (Umgebung 1) und, falls vorhanden, das unmittelbar benachbarte Atom oder die unmittelbar benachbarten Atome (Umgebung 2) jedes der unmittelbar benachbarten Atome des Herds gebildet ist,
- Mittel (81, 89), um eine Fragmentedatei zu erzeugen, die für jedes berechnete Fragment der Formeln der Basisdatei die Liste der Bezugsnummern der dieses Fragment enthaltenden Formeln enthält,
- Mittel (81) zur Extrahierung von Bezugsnummern-Listen möglicher Formeln, die mindestens ein Fragment der gesuchten Markush-Formel enthalten, ausgehend von der Fragmentedatei, wobei die Mittel vorgesehen sind, um eine Liste zu erzeugen, "die von Fragmenten" einer optimierten Länge "abgeleitet ist", gebildet durch die Markush-Nummern, die eine durch den Benutzer bestimmte, optimierte Anzahl von mit der gesuchten Markush-Formel gemeinsamen Fragmenten aufweisen,
- und Mittel (81) zum Vergleich der "Vorauswahl"-Nummer der gesuchten Markush-Formel mit den "Vorauswahl"-Nummern der Formeln der Liste, "die von Fragmenten" einer optimierten Länge und ausgehend von diesem Vergleich "abgeleitet ist", zur Bildung einer abschließenden Liste, die aus den Bezugsnummern der Formeln gebildet ist, die eine "Vorauswahl"-Nummer aufweisen, die mit derjenigen der gesuchten Markush-Formel identisch ist.

13. System nach Anspruch 12,
dadurch gekennzeichnet, daß es ferner Mittel zum Knotenzu-Knoten-Vergleich der Formeln der abschließenden Liste mit der gesuchten Markush-Formel und zur Extrahierung der Bezugspunkte bzw. Referenzen der Markush-Formel oder der Markush-Formeln aufweist, die gegebenenfalls durch diesen Vergleich erhalten wird bzw. werden.

14. System nach einem der Ansprüche 12 und 13,
dadurch gekennzeichnet, daß die Mittel zur Erzeugung einer Datei chemischer Formeln ausgehend von den Markush-Formeln in Form von Konnektivitätstabellen Mittel zur Bildung einer Vorauswahl-Nummer in Form einer Kette von n Bits aufweisen, von denen jedes Bit einem Typzeichen entspricht, das von der Definitionstabelle bestimmt ist, die zuvor aus den n Typzeichen (Tabelle VII) gebildet wurde.

## Claims

1. A method of storing and searching Markush formulae (1), the method being characterized in that:
a) a base file (14) of chemical formulae is formed from said Markush formulae, in which each Markush formula is stored in the form of a connectivity table (TC₁, TC₂, ... TCₙ) comprising a reference number (NOFM₁, NOFM₂, ... NOFMₙ) of said stored formula, and a "preselection" number (Nps₁, Nps₂, ... Npsₙ) synthetically describing predetermined typical features of said formula, defined according to a previously constructed definition table of said typical features (Table VII);
b) for each formula of the base file (14), and for the searched-for formula (3) stored in identical form to the formulae of the file, the list of all the focal nodes or atoms comprising at least two immediately adjacent nodes or atoms is determined and, a "fragment" being defined as the part of a formula structure constituted by a focus, its immediately adjacent atoms (environment 1) and, if they exist, the immediately adjacent atom(s) of the focus (environment 2) of each of the immediately adjacent atoms of the focus, for each node of said list, all the possible parts, or fragments, of said formula resulting from said node are calculated;
c) a fragment file (18) is formed comprising, for each calculated fragment of the formulae of the base file, the list of reference numbers of said formulae comprising said fragment:
d) for a first fragment of the searched-for Markush formula, a first list of reference numbers of possible formulae comprising said fragment of said searched-for Markush formula is extracted from said fragment file (18):
e) then, where necessary, and for a second fragment of the searched-for formula, a second list of reference numbers of possible formulae comprising said second fragment is extracted from said fragment file (18); said second list is compared with the first list obtained for the first fragment, in order to form a "fragment outcome" third list, constituted by numbers common to the first two lists.
and the operation is reiterated as often as it is considered necessary by comparing each new list of numbers of possible formulae corresponding to a new fragment with the list constituted by the numbers common to all the previous lists, in order to form a "fragment outcome" list of optimized length;
f) the "preselection" number of the searched-for Markush formula is compared with the "preselection" numbers of the formulae of the "fragment outcome" list of optimized length, and a final list is extracted therefrom, formed by the reference numbers of formulae having a "preselection" number identical to that of the searched-for Markush formula.

2. A method of storing and searching Markush formulae (1), according to claim 1, the method being characterized in that, starting from a data base (6) comprising the base file (14) of chemical formulae formed from Markush formulae (1), and the fragment file (18) comprising the lists of reference numbers of the formulae associated to each fragment of the formulae of the base file (14), the searched-for Markush formulae (3) is stored in similar form to the formulae of the data base (6), i.e. in the form of a connectivity table (TCᵢ) with a preselection number (Npsi) of said searched-for formula.

3. A method according to claim 1, enabling an operator to store and search Markush formulae (1), the method being characterized in that each Markush formula of the base file (14) is stored in a computer system by the operator,
in that the list of all the focal nodes or atoms comprising at least two immediately adjacent nodes or atoms is determined automatically for each formula of the base file (14) and for the searched-for formula (3) stored by the operator in identical form to the formulae of the file,
in that all the possible parts, or fragments, of said formula resulting from said node are calculated automatically for each node of said list,
in that the fragment file (18) is formed automatically,
in that the second list of reference numbers of the possible formulae comprising said second fragment is extracted following a command by the operator and is automatically compared with the first list obtained for the first fragment,
in that the operation is reiterated under manual command of the operator by automatically comparing each new list of numbers of possible formulae corresponding to a new fragment with the list constituted by the numbers common to all the previous lists, in order automatically to form a "fragment outcome" list of optimized length, and
in that following a manual command by the operator, the "preselection" number of the searched-for Markush formula is automatically compared with the "preselection" numbers of the formulae of the "fragment outcome" list of optimized length, the final list being automatically extracted therefrom.

4. A method according to claims 2 and 3, enabling a user to store and search Markush formulae (1) in a computer system, the method being characterized in that each Markush formula is stored in the computer system in the form of connectivity tables (TCᵢ),
in that the user stores the searched-for Markush formula (3) in the computer system in similar form to the formulae of the data base (6),
in that the computer system automatically determines the list of nodes for the searched-for formula (3), and calculates all the fragments,
in that the computer system automatically extracts the first list of reference numbers from said fragment file (18),
in that the computer system automatically extracts the second list of reference numbers from said fragment file (18) and compares it with the first list obtained for the first fragment, in order to form the third list,
in that the computer system reiterates the operation initiated by the manual command of the user, as often as the user considers it necessary, by automatically comparing each new list with the list constituted by the numbers common to all the previous lists, and
in that, following a manual command by the operator, the computer system automatically compares the "preselection" number with the "preselection" numbers of the formulae of the "fragment outcome" list and extracts the final list therefrom.

5. A method according to claims 2 and 3, enabling a user to store and search Markush formulae (1) in a computer system, the method being characterized in that, starting from the data base (6) of the computer system, the user inputs, at least in part, the searched-for Markush formula (3) in a graphic form determined by the computer system;
in that the computer system reads said graphic representation and transforms it in order to store it;
in that the operation of forming the third list is reiterated by the computer system which automatically compares each new list with the list constituted by the numbers common to all the previous lists, until said comparison no longer allows the number of formulae of the "fragment outcome" list to be significantly reduced in order to form a fragment outcome list of optimized length;
in that the computer system, in automatic mode, or the user, in manual mode, then controls the step where said computer system compares the "preselection" number with the "preselection" numbers of the formulae of the "fragment outcome" list, and extracts the final list therefrom; and
in that the computer system extracts, one by one, at the request of the user, and where necessary, the formulae retained in said final list, by transforming them in order to represent them in the form of matching graphic images suitable for enabling optimized comparison with the determined graphic form of the searched-for formula.

6. A method according to any preceding claim, characterized in that a node-by-node comparison of the formulae of said final list with the searched-for Markush formula (3) is again performed, and the references of any Markush formula obtained by said comparison are extracted.

7. A method according to any preceding claim, characterized in that a set of attributes is allocated to some of the atoms or groups of atoms of the Markush formulae of the interrogated base file (14), and, where necessary, to the searched-for Markush formula (3), and said base file of Markush formulae is searched only for the formulae including a structure provided with the attribute(s) specifically allocated to the searched-for formula.

8. A method according to any preceding claim, characterized in that the Markush formulae constituted by graphs are stored using, when necessary at the nodes of said graphs, false atoms representing generic terms and/or invariant sets of atoms to simplify the representations of said formulae.

9. A method according to any preceding claim, characterized in that the Markush formulae are stored in the base file (14) and said base file is interrogated in order to search for a specific Markush formula, in essentially graphic form, by means of a "mouse" type interface.

10. A method according to any preceding claim, characterized in that the "preselection" number (Npsi), synthetically describing predetermined typical features of a Markush formula, is constituted in the form of a chain of n bits, of which each bit corresponds to a typical feature determined from the previously constructed definition table of said n typical features (Table VII).

11. A method according to any preceding claim, characterized in that the conductivity tables representing the Markush formulae are constituted by "chest-of-drawers" connectivity tables comprising:
four tables giving the relationships between the various groups of atoms or "daughter" graphs of the structure, constituted by a first table (LIRAT) identifying the atoms to which said groups are attached;
a second table (NVAL) giving the numbers of values for each of said groups;
a third table (IAT) giving the start values of said groups; and
a fourth table (IFR) giving the addresses per group towards the start value table (IAT).

12. A computer system for implementing the method of storing and searching Markush formulae, according to any preceding claim, the system comprising a central processing unit (81), working and storage memory (82, 88), input storage (84, 85, 87) for inputting Markush formulae in graphical and/or alphanumeric form, and means for restoring data (86, 87), said system being characterized in that it comprises:
generator means (81, 89) for generating a base file (14) of chemical formulae from said Markush formulae in the form of connectivity tables (TC₁), each formula comprising a reference number (NOFM₁), and a "preselection" number (Npsi) describing the typical features of said formula defined by a definition table (Table VII) which has previously been stored in a memory (88) of the system;
generator means (81, 89) for generating, for each stored or searched-for formula, the list of all the focal nodes or atoms comprising at least two immediately adjacent nodes or atoms;
calculating means (89) for calculating, for each node of said list, all possible parts, called fragments, of said formula, resulting from said node, a fragment being defined as the part of a formula structure comprising a focus, its immediately adjacent atoms (environment 1) and, if they exist, the immediately adjacent atom(s) (environment 2) of each of the immediately adjacent atoms of the focus;
generator means (81, 89) for generating a fragment file comprising, for each calculated fragment of the formulae of the base file, the list of reference numbers of the formulae comprising said fragment;
extraction means (81) for extracting from said fragment file, lists of reference numbers of possible formulae comprising at least one fragment of said searched-for Markush formula, said means being provided in order to generate a "fragment outcome" list of optimized length constituted by the Markush numbers including an optimized number, determined by the user, of fragments common to the searched-for Markush formula; and
comparison means (81) for comparing the "preselection" number of the searched-for Markush formula with the "preselection" numbers of the formulae of the "fragment outcome" list of optimized length, and from said comparison, the constitution of a final list formed by the reference numbers of formulae having a "preselection" number identical to that of the searched-for Markush formula.

13. A system according to claim 12, characterized in that it further comprises comparison means for node-by-node comparison of the formulae of said final list with the searched-for Markush formula, and extraction means for extracting the references of any Markush formula obtained by said comparison.

14. A system according to claim 12 or 13, characterized in that the means for generating a file of chemical formulae from said Markush formulae in the form of a connectivity table comprise means for constituting a preselection number in the form of a chain of n bits, of which each bit corresponds to a typical feature determined from the previously constructed definition table of said n typical features (Table VII).
